# EUROPÄISCHE PATENTANMELDUNG

(11) **EP 1 254 963 A1**
(43) Veröffentlichungstag der Anmeldung: **06.11.2002**
(21) Anmeldenummer: 01110840.4
(22) Anmeldetag: 04.05.2001
(51) Int. Cl.: C12Q 1/68

(54) **Nukleinsäuresequenzen und Verfahren zum gattungs-, gruppen-, spezies- und subspeziesspezifischen Nachweis von Mycobakterien**

(71) Anmelder: BioChip Technologies GmbH, 79108 Freiburg i. Br. (DE)
(72) Erfinder: Rolfs, Arndt, 18055 Rostock (DE); Tiemer, Bettina, 23564 Lübeck (DE)
(74) Vertreter: Teipel, Susanne, Dr.

(57) **Zusammenfassung**

Ein Verfahren zum Nachweis von Mycobakterien, beruhend auf der 23S/5S-Spacerregion von Mycobakterien hat den Nachweis der möglichen gattungsspezifischen, gruppenspezifischen, speziesspezifischen und subspeziesspezifischen Amplifikation.

## Beschreibung

Gegenstand der Erfindung sind Verfahren zur Amplifikation von Nukleinsäuren der Gattung Mycobacterium und Verfahren zum gattungs- und speziesspezifischen Nachweis von Bakterien der Gattung Mycobacterium sowie dafür geeignete Reagenzien.

Seit Bereitstellung von Verfahren zur Amplifikation von Nukleinsäuren, z. B. der Polymerase Chain Reaction (PCR), haben sich Verfahren zum Nachweis von Infektionen und genetischen Zuständen in einigen Fällen als sehr hilfreich in der Klinischen Diagnostik erwiesen. So ist es in einigen Fällen möglich, beispielsweise ganz spezifisch einzelne Spezies, aber auch ganze Genera von Organismen nachzuweisen. Für gruppen- und speziesspezifische Nachweise von Mycobakterien ist insbesondere die 16S-Region beschrieben (EP-A-O 528 306). Leider erlaubt diese Region keine gattungsspezifische Amplifikation, so daß für jede Gruppe oder Spezies eigene Primer gefunden und verwendet werden müssen. Dies erhöht die Komplexität des Nachweises oder erfordert sogar die Amplifikation in getrennten Ansätzen, was die erforderliche Probenmenge erhöht. In der EP-96106728.7 ist ein Verfahren zum Nachweis von Legionellen beschrieben, das auf der Amplifikation von Nukleotidsequenzen aus der 23S/5S-Spacerregion von Legionellen beruht.

In Microbiology (1994), 140, 123-132 und 1763-1773 sind Nukleotidsequenzen angegeben, die der 16S rRNA und Spacerregionen von langsam wachsenden Mycobakterien zugeordnet sein sollen.

Aufgabe der vorliegenden Erfindung war die Bereitstellung von Reagenzien, die den Nachweis von Mycobakterien zuverlässiger machen. Eine weitere Aufgabe war es, potentiell variablere Mycobakteriennachweise, die auch den spezifischen Subspeziesnachweis ermöglichen, zur Verfügung zu stellen.

Gegenstand der Erfindung ist daher in einem ersten Aspekt ein Verfahren zur Amplifikation von Nukleinsäuren der Gattung Mycobacterium, dadurch gekennzeichnet, daß eine Nukleinsäure amplifiziert wird, die eine Länge von mehr als 90 Nukleotiden hat und die zu mehr als 75 % homolog oder komplementär zu aufeinanderfolgenden Basen der SEQ.ID.NO.1 ist.

Ein weiterer Gegenstand der Erfindung sind Nachweisverfahren unter Verwendung des oben genannten Amplifikationsverfahrens.

Ein weiterer Gegenstand der Erfindung ist ein Verfahren zum Nachweis von Bakterien oder Gattung Mycobacterium unter Verwendung einer Nukleinsäuresonde, welche eine mindestens 15 aufeinanderfolgende Basen lange Sequenz aufweist, die zu mindestens 90 % homolog zu einem Teil der SEQ.ID.NO.1 ist oder die zu mindestens 90 % komplementär zu einem Teil der SEQ.ID.NO.1 ist.

Kern der Erfindung ist, daß Sequenzen auf dem Mycobakterien-Genom lokalisiert wurden, die das Entwerfen von Nukleinsäuresonden erlauben, die zum gruppen-, spezies- und subspeciesspezifischen Nachweis innerhalb der Gattung Mycobacterium verwendet werden können.

Unter Amplifikation wird die Erhöhung der Konzentration der in einem Reaktionsgemisch vorhandenen Nukleinsäuren oder Teilen davon verstanden. Beispiele für solche Amplifikationsverfahren sind die Polymerase-Kettenreaktion gemäß EP-B-0 201 184, das sogenannte NASBA-Vefahren gemäß EP-A-0 329 822 sowie davon abgeleitete Verfahren.

Bei dem Verfahren der EP-A-0 201 184 wird die zu amplifizierende Nukleinsäure, die einzelsträngig vorliegt oder einzelsträngig gemacht wird, mit einem molaren Überschuß zweier Oligonukleotid-Primer unter Hybridisierungsbedingungen und in Gegenwart eines induzierenden Agens für die Polymerisation und Nukleotiden behandelt, wobei die Primer so gewählt werden, daß für jeden Strang ein zum Nukleinsäurestrang komplementäres Verlängerungsprodukt des betreffenden Primers synthetisiert wird, und daß ein Verlängerungsprodukt eines Primers, wenn es von seinem Komplement getrennt ist, als Matrize zur Synthese eines Verlängerungsprodukts des anderen Primers dienen kann, Trennen der Verlängerungsprodukte von den Matrizen, an denen sie synthetisiert wurden, und Verwendung der gebildeten Verlängerungsprodukte zur erneuten Umsetzung mit den Primern. Durch die zyklische Wiederholung der Schritte ergibt sich eine theoretisch exponentielle Vermehrung von Nukleinsäuresequenz, die innerhalb der äußeren Hybridisierungspositionen der Primer liegt. Unter einem Amplifikat wird daher bevorzugt eine Kopie der nachzuweisenden Nukleinsäuren in einem bestimmten, durch die Auswahl der Hybridisierungspositionen der Primer festgelegten Teilbereich verstanden.

Bei dem Verfahren gemäß EP-A-0 329 822 wird eine Primerkonstruktion zur Erzeugung einer doppelsträngigen Nukleinsäure verwendet, in der die zu amplifizierende Nukleinsäuresequenz funktionell an einen Promotor gebunden ist. Hierzu weist einer der Primer mindestens einen Strang einer Promotorregion auf. Der intermediär gebildete Transkriptionskomplex wird Bedingungen unterworfen, unter denen unter der Kontrolle des Promotors Ribonukleinsäuren unter Verwendung der zu amplifizierenden Nukleinsäure als Matrize gebildet werden. Die gebildeten Ribonukleinsäuren werden erneut zur Bildung jeweils eines neuen transkribierbaren Nukleinsäurekomplexes verwendet. Ein Vorteil dieses Systems ist, daß es isotherm geführt werden kann.

Unter Multiplex-PCR wird ein Verfahren gemäß EP-A-0 364 255 verstanden. Nach diesem Verfahren werden in einer Eintopfreaktion durch geeignete Anordnung der Hybridisierungspositionen einer Vielzahl von Primern bestimmte Fragmente von Nukleinsäuren amplifiziert, die meist unterschiedliche Länge und Position im Genom aufweisen.

Bei dem erfindungsgemäßen Verfahren handelt es sich um eine spezielle Ausführungsform der sogenannten Hybridisierungstests, die in ihren Grundzügen dem Fachmann auf dem Gebiet der Nukleinsäurediagnostik bekannt sind. Soweit experimentelle Details im folgenden nicht ausgeführt sind, wird dazu vollinhaltlich auf "Nucleic acid hybridisation", Herausgeber B.D. Hames und S.J. Higgins, IRL Press, 1986, insbesondere in den Kapiteln 1 (Hybridisation Strategy), 3 (Quantitative Analysis of Solution Hybridisation) und 4 (Quantitative Filter Hybridisation), Current Protocols in Molecular Biology, Edt. F.M.

Ausubel et al., J. Wiley and Son, 1987, insbesondere 2.9.1. - 2.9.10 und Molecular Cloning, Edt. J. Sambrook et al., CSH, 1989, insbesondere 9.4.7 - 9.5. 8, Bezug genommen. Dazu gehören insbesondere die bekannten Methoden zur Herstellung von markierten Nukleosidtriphosphaten, wie sie auch in EP-A-0 329 474 beschrieben sind, die chemische Synthese von modifizierten und unmodifizierten Oligonukleotiden, die Spaltung von Nukleinsäuren mittels Restriktionsenzymen, die Auswahl von Hybridisierungsbedingungen, durch welche eine Spezifität erreicht werden kann, die vom Ausmaß der Homologie zwischen den zu hybridisierenden Nukleinsäuren, deren GC-Gehalt und deren Länge abhängt, sowie die Bildung von Nukleinsäuren aus Nukleosidtriphosphaten mit Hilfe von Polymerasen, gegebenenfalls unter Verwendung von sogenannten Primern.

Eine Markierung im Sinne der vorliegenden Erfindung besteht aus einer direkt oder indirekt nachweisbaren Gruppe. Direkt nachweisbare Gruppen sind beispielsweise radioaktive (³²P), farbige oder fluoreszierende Gruppen oder Metallatome. Indirekt nachweisbare Gruppen sind beispielsweise immunologisch oder enzymatisch wirksame Verbindungen, wie Antikörper, Antigene, Haptene oder Enzyme oder enzymatisch aktive Teilenzyme. Diese werden in einer nachfolgenden Reaktion oder Reaktionssequenz detektiert. Besonders bevorzugt sind Haptene, da mit ihnen markierte Nukleosidtriphosphate (rNTP oder dNTP) im allgemeinen besonders gut als Substrate von (RNS- bzw. DNS-) Polymerasen einsetzbar sind und eine anschließende Reaktion mit einem markierten Antikörper gegen das Hapten oder das haptenisierte Nukleosid leicht vorgenommen werden kann. Solche Nukleosidtriphosphate sind beispielsweise Brom-Nukleosidtriphposphate. Als besonders geeignet haben sich die in EP-A-0 324 474 genannten Steroide und deren Detektion erwiesen. Für deren Inkorporation in Nukleinsäuren wird hiermit auf die EP-A-0 324 474 verwiesen.

Unter den Nukleinsäuren, die zur Verwendung des erfindungsgemäßen Amplifikationsund Nachweisverfahrens für Mycobakterien verwendet werden können, werden insbesondere genomische Nukleinsäuren verstanden. Genomische Nukleinsäuren enthalten unter anderem auch die Gene, welche für die ribosomale RNS (rRNS) codieren sowie eine Reihe von Spacersequenzen. Diese werden nach der Größe der rRNS, für die sie codieren, benannt. In Mycobakteriengenomen sind in dieser Reihenfolge das 16S-rRNS-Gen, das 23S-rRNS-Gen und das 5S-rRNS-Gen angeordnet. Diese Gene sind durch sogenannte Spacerregionen (16S/23S- und 23S/5S-Spacer) voneinander getrennt.

SEQ.ID.NO.1 offenbart eine Mehrheitssequenz auf Basis der 23S/5S-Spacer-Sequenz ausgewählter Mycobakterien der Tabelle 1; SEQ.ID.NO.2 offenbart die Mehrheitssequenz auf Basis der partiellen 3'-Region der 23S-Sequenz der Mykobakterien; SEQ.ID.NO.3 offenbart die Mehrheitssequenz auf Basis der partiellen 5'-Region der 5S-Sequenz der Mykobakterien und SEQ.ID.NO.4 die Mehrheitssequenz der intergenischen Spacerregion zwischen 23S und 5S für die Mykobakterien. Dabei wird unter einer Mehrheitssequenz die Nukleotidsequenz verstanden, die die Nukleotide einer bestimmten Gruppe von Organismen enthält, die bei der Mehrzahl der untersuchten Organismen an dieser Position vorzufinden sind (gemäß Clustal-Methode). Abweichungen der Sequenzen einzelner Organismen von dieser Mehrheitssequenz sind ein Anzeichen für eine Spezifität der Sequenz gegenüber der Mehrheit der Organismen der Gruppe. Im Sinne der vorliegenden Erfindung soll der Begriff Nukleinsäuresonde nicht im Hinblick auf die Funktion einschränkend betrachtet werden. Die oben genannten Eigenschaften, insbesondere die Spezifität, sind maßgebend. Insbesondere sollen zu den Nukleinsäuresonden die sogenannten Probes oder Nachweissonden gezählt werden, deren Hybridisierung mit den in der Probe vorhandenen Mycobakteriennukleinsäuren als Maß für die Anwesenheit des Bakteriums verwendet werden kann. Dazu kann die Nukleinsäuresonde bevorzugt weitere Teile enthalten, die den Nachweis oder die Amplifikation nicht wesentlich nachteilig beeinflussen. Zu solchen Molekülteilen gehören beispielsweise Markierungen zum Nachweis der Sonden bzw. der Hybride, die diese Sonde enthalten, Gruppen, welche eine Immobilisierung der Nukleinsäuresonde an eine feste Phase erlauben, feste Phasen als solche (z. B. Beads oder Oberflächen, z. B. von Reagenz- oder Detektionsgefäßen) oder weitere, nicht mit Legionella-Sequenzen interferierende Nukleotidsequenzen. Die Ausgestaltung der Nukleinsäuresonde richtet sich daher insbesondere nach der Art, wie der Nachweis der Legionella-Nukleinsäuren geführt werden soll. Ein Fachmann kann auf einfache Weise die erfindungsgemäßen Sequenzen mit solchen zusätzlichen Molekülteilen versehen.

Unter einer Nukleinsäuresonde werden auch die sogenannten Primer verstanden, welche nach Hybridisierung mit Mycobakterien-Nukleinsäuren unter Verwendung eines Enzyms verlängert werden können. Typische Verlängerungsreaktionen sind beispielsweise die Anhängung von Mononukleosidtriphosphateinheiten durch DNS-Polymerasen (z. B. E. coli DNS-Polymerase, Klenow-Fragment oder Thermus-aquaticus-Polymerase) unter Verwendung der Mycobakterien-Nukleinsäuren als Matrize. In diesem Fall wird mit den bislang bekannten Enzymen das 3'-Ende des Primers verlängert. Eine weitere Verlängerungsreaktion ist die Ligase-Reaktion, bei der die Nukleinsäuresonde mit einem weiteren Oligonukleotid enzymatisch verknüpft wird (z. B. EP-A-0 320 308). Zwischen der Polymerase- und der Ligasereaktion sind Mischformen bekannt (z. B. WO 90/01069). Nukleinsäuresonden enthalten eine festgelegte Aufeinanderfolge von Basen und sind in der Lage, mit natürlichen Nukleinsäuren Hybride aufgrund von Basen-Basen-Wechselwirkungen zu bilden. Zu den Nukleinsäuresonden gehören natürliche und artifizielle Nukleinsäuren. Die artifiziellen Nukleinsäuren unterscheiden sich von den natürlichen Nukleinsäuren dadurch, daß sie entweder im Basenteil (z. B. durch Verwendung von Basenanaloga, z. B. 7-deaza-dGTP) oder im Grundgerüst (Ersatz der Zucker- oder/und Phosphateinheiten durch andere chemische Molekülteile, z. B. Peptide) modifiziert sind. Derartige künstliche Nukleinsäuren sind beispielsweise auch die in WO 92/20702 beschriebenen Peptidnukleinsäuren (PNA). Wesentlich für die korrekte Funktionsweise der Nukleinsäuresonden ist die spezifische Basenpaarung, wodurch die Selektivität der Sonden erreicht wird. Besonders bevorzugt besteht eine Nukleinsäuresonde aus Desoxyribonukleinsäure (DNS).

Unter einer für Mycobakterien gattungsspezifischen Nukleinsäuresonde wird eine Nukleinsäure verstanden, die mit Nukleinsäuren der Bakterien der Gattung Mycobacterium hybridisiert, nicht jedoch mit Nukleinsäuren anderer Gattungen. Besonders bevorzugt hybridisierenden diese Nukleinsäuren mit allen möglichen Spezies der Gattung Mycobakterium. Diese gattungsspezifischen Sequenzen liegen bevorzugt in Bereichen, die sich direkt an die Sequenz der SEQ.ID.NO.1 anschließen, das heißt in der 23S und der 5S Region, bevorzugt innerhalb von 120 Basen vom 5'-Edne bzw. 50 Basen vom 3'-Ende der SEQ.ID.NO.1. Besonders bevorzugte gattungsspezifische Sequenzen sind Sequenzen, die zu mindestens 90 %, bevorzugt zu 100 %, homolog oder komplementär zu mindestens 10 aufeinanderfolgenden Nukleotiden der SEQ.ID.NO 2 (3'- Ende der 23S-Region) oder 3 (5'-Ende der 5S-Region) sind.

**Tab.1:**

| Verwendete Mykobakterium-Species bzw. Subspezies | | | | |
|---|---|---|---|---|
| **Spezies** | **Abkürzung** | **Hinterlegungsnr.** | **Amplifikatlänge** | **SEQ.ID.No.** |
| M. africanum | AFRICA | | 263bp | 11 |
| M. africanum | AFR25420 | ATCC 25420 | 263bp | 10 |
| M. avium | AVI15769 | ATCC 15769 | 249bp | 12 |
| M. avium | AVI35712 | ATCC 35712 | 249bp | 14 |
| M. avium | AVI35718 | ATCC 35718 | 249bp | 15 |
| M. avium | AVI35771 | ATCC 35771 | 249bp | 8 |
| M. avium | AVI19698 | ATCC 19698 | 249bp | 13 |
| M. avium | AVI35768 | ATCC 35768 | 249bp | 16 |
| M. avium | AVIUM1 | Serovar 1 | 249bp | 17 |
| M. avium | AVIUM16 | Serovar 16 | 248bp | 18 |
| M. avium | AVIUM19 | Serovar 19 | 239bp | 19 |
| M. avium | AVIUM26 | Serovar 26 | 248bp | 20 |
| M. avium | AVIUM28 | Serovar 28 | 248bp | 21 |
| M. avium* | AVIUM4 | Serovar 4 | 249bp | 22 |
| M. avium | AVIUM5 | Serovar 5 | 249bp | 23 |
| M. avium | AVIUM6 | Serovar 6 | 249bp | 24 |
| M. avium | AVIUM7 | Serovar 7 | 239bp | 25 |
| M. avium | AVIUM9 | Serovar 9 | 249bp | 26 |
| M. bovis | BOV27289 | ATCC 27289 | 263bp | 7 |
| M. bovis | BOVIS1 | Serovar 1 | 263bp | 27 |
| M. bovis | BOVIS2 | Serovar 2 | 263bp | 28 |
| M. bovis | BOVIS4 | Serovar 4 | 263bp | 29 |
| M. fortuitum | FOR7360 | | 307bp | 30 |
| M. gordonae | GORDON | | 245bp | 31 |
| M. gordonae | GOR14470 | ATCC 14470 | 245bp | 32 |
| M. intrazellulare | INTRA | | 248bp | 41 |
| M. intrazellulare | INT35761 | ATCC 35761 | 248bp | 34 |
| M. intrazellulare | INT35762 | ATCC 35762 | 248bp | 35 |
| M. intracellulare | INT35763 | ATCC 35763 | 248bp | 9 |
| M. intracellulare | INT35764 | ATCC 35764 | 248bp | 36 |
| M. intrazellulare | INT35765 | ATCC 35765 | 249bp | 37 |
| M. intrazellulare | INT35766 | ATCC 35766 | 249bp | 38 |
| M. intrazellulare | INT35772 | ATCC 35772 | 239bp | 6 |
| M. intrazellulare | INT35774 | ATCC 35774 | 249bp | 39 |
| M. intracellulare | INT35847 | ATCC 35847 | 240bp | 40 |
| M. intracellulare | INT13950 | ATCC 13950 | 248bp | 33 |
| M. kansasii | KANB180 | | 240bp | 43 |
| M. kansasii | KAN12478 | ATCC 12478 | 240bp | 42 |
| M. phlei | PHLEI | | 251bp | 50 |
| M. smegmatis | SMEGM | | 305bp | 51 |
| M. szulgai | SZULGAI | | 246bp | 53 |
| M. szulgai | SZU35799 | ATCC 35799 | 245bp | 52 |
| M. terrae | TERRAE | | 263bp | 54 |
| M. tuberculosis | MTB27294 | ATCC 27294 | 263bp | 5 |
| M. tuberculosis | MTB-P02 | Patientenisolat | 263bp | 44 |
| M. tuberculosis | MTB-P03 | Patientenisolat | 263bp | 45 |
| M. tuberculosis* | TB-P05 | Patientenisolat | 263bp | 46 |
| M. tuberculosis | MTB-P08 | Patientenisolat | 263bp | 47 |
| M. tuberculosis* | TB-P10 | Patientenisolat | 263bp | 48 |
| M. tuberculosis | MTB-P14 | Patientenisolat | 263bp | 49 |
| M. xenopi | XENOPI | | 263bp | 55 |
| Liste der mittels der Primer MCONl/MCON2c hergestellten und analysierten Mykobakterien, die die Grundlage zur Herstellung der Mehrheitssequenz SEQ.ID:Nol bildeten; in die Alignmentanalyse gingen die mit "*" markierten Sequenzen nicht ein. | | | | |

Unter **gruppenspezifischen** Nukleinsäuresonden werden solche Sonden verstanden, die mit allen Spezies/Subtypen/Serovaren einer Mycobakteriengruppe unter denselben Stringensbedingungen hybridisieren. Bevorzugte Mycobakteriengruppen sind die Gruppen des MTB-Komplexes (enthaltend die Spezies M. tuberculosis, M. microti, M. bovis und M. africanum), des Komplexes gebildet aus mindestens 2 der Mitglieder des MTB-Komplexes, insbesondere aus M. tuberculosis, M. bovis und M. africanum und des MAI-Komplexes (enthaltend die Spezies M. avium und M. intrazellulare). Innerhalb der Spacerregion sind bestimmte Bereiche zur Auswahl von gruppenspezifischen Sequenzen insbesondere für Nachweissonden besonders bevorzugt.

Besonders bevorzugte Bereiche liegen zwischen den Positionen 109 und 141 sowie 245 und 298 der SEQ.ID.NO.1 bzw. zwischen den Positionen 106 und 172 sowie 214 und 245 der SEQ.ID.NO.5. Besonders bevorzugt schließt die Sequenz der Sonde die Sequenz von Position 145 bis 202 der SEQ.ID. NO.5, SEQ.ID.NO.44-49 und SEQ.ID.NO.55 für M. tuberculosis und von Position 150 bis 190 der SEQ.ID.NO.8 und SEQ.ID.NO.12-26 für M. avium intracellulare ein.

Unter **speziesspezifischen** Nukleinsäuresonden werden Nukleinsäuren verstanden, die mit allen Subtypen/Serovaren einer Mycobakterienspezies unter denselben Stringensbedingungen hybridisieren. Innerhalb der SEQ.ID.NO.1 sind bestimmt Bereiche zur Auswahl von speziesspezifischen Sequenzen insbesondere für Nachweissonden besonders bevorzugt. Besonders bevorzugte Bereiche liegen zwischen den Positionen 178 und 213 der SEQ.ID.NO. 1.

**Subspeziesspezifische** Nukleinsäuresonden sind solche, die mit einem oder mehreren Subtypen/Serovaren einer Mycobakterienspezies, nicht jedoch mit anderen Subtypen/Serovaren dieser Spezies unter denselben Stringensbedingungen hybridisieren. Innerhalb der SEQ.ID.NO.1 sind bestimmte Bereiche zur Auswahl von subspeziesspezifischen Sequenzen insbesondere für Nachweissonden besonders bevorzugt. Besonders bevorzugte Bereiche liegen zwischen den Positionen 142 und 160 der SEQ.ID.NO. 1 bzw. zwischen den Positionen 150 und 190 der SEQ.ID.NO.8 und SEQ.ID.NO.12-26.

### Kurze Beschreibung der Zeichnungen

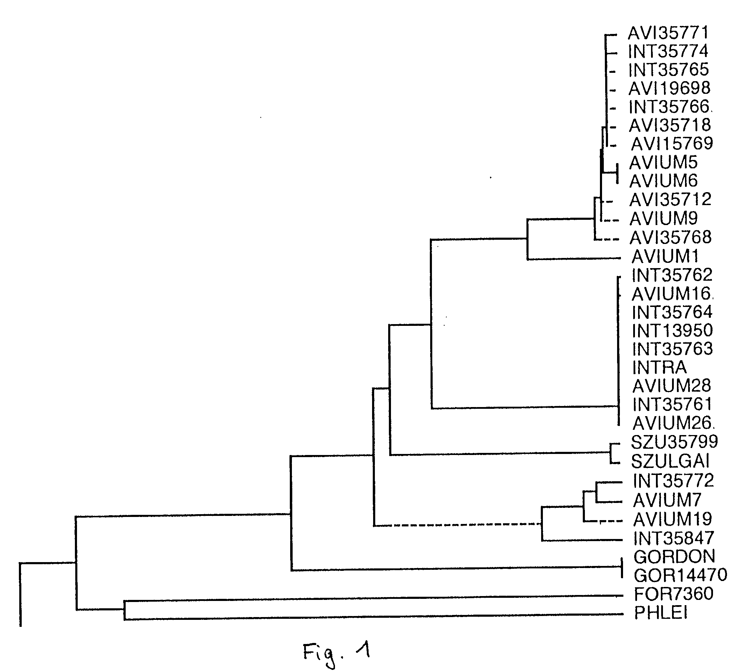

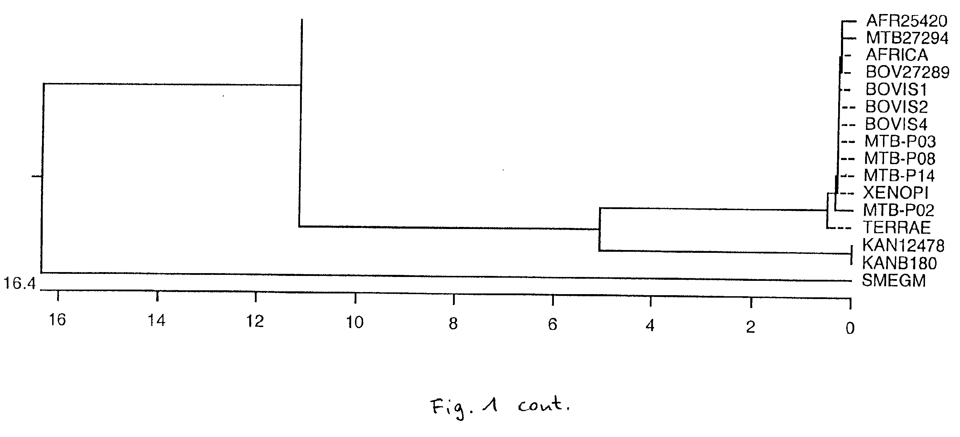

**Figur 1:** Phylogenetischer Stammbaum einiger untersuchter Mycobakterien (Phylogenetic tree of PATENT1.meg); die Bezeichungen sind konsistent mit den Bezeichnungen der Tabelle 1.

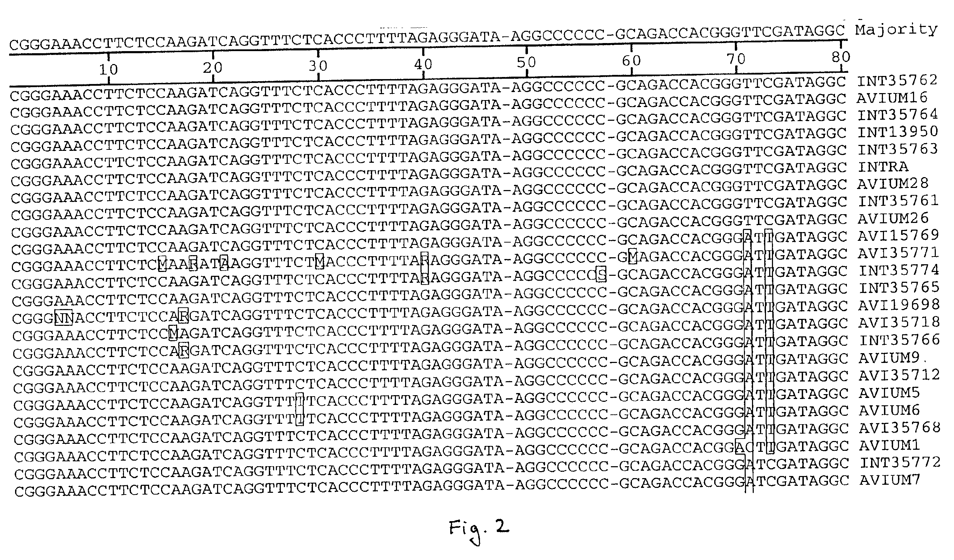

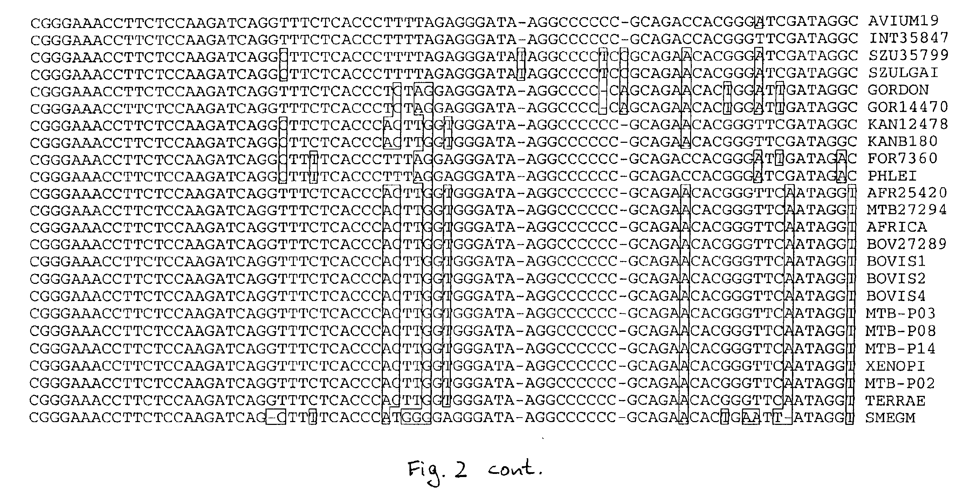

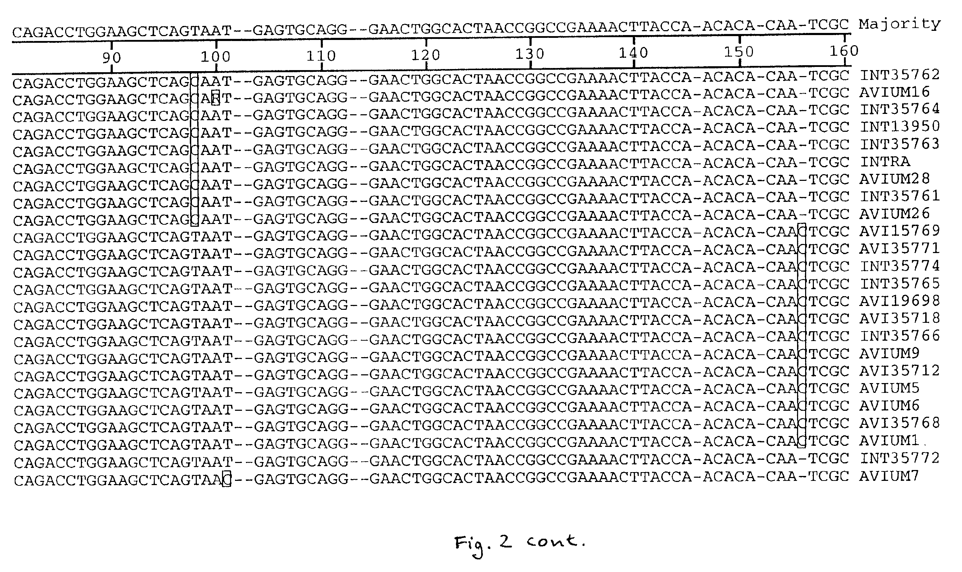

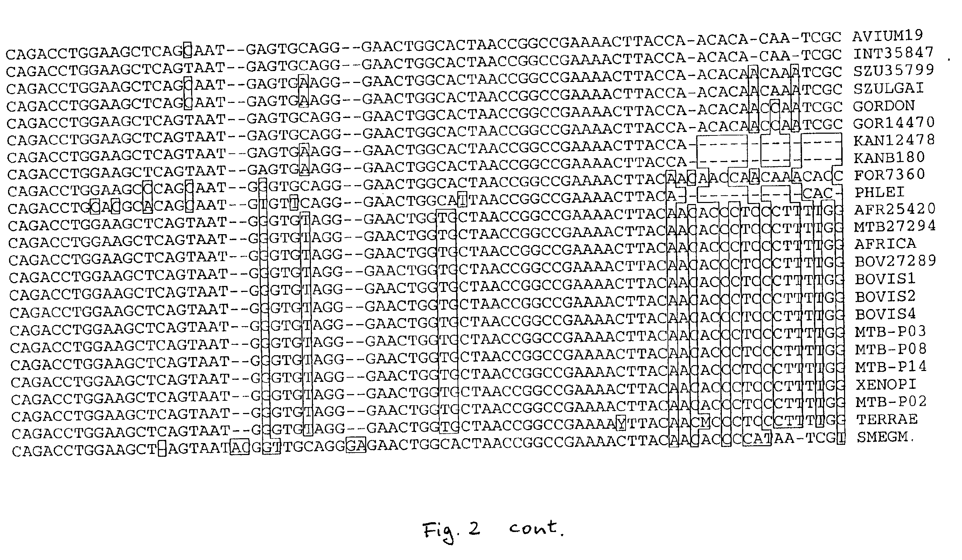

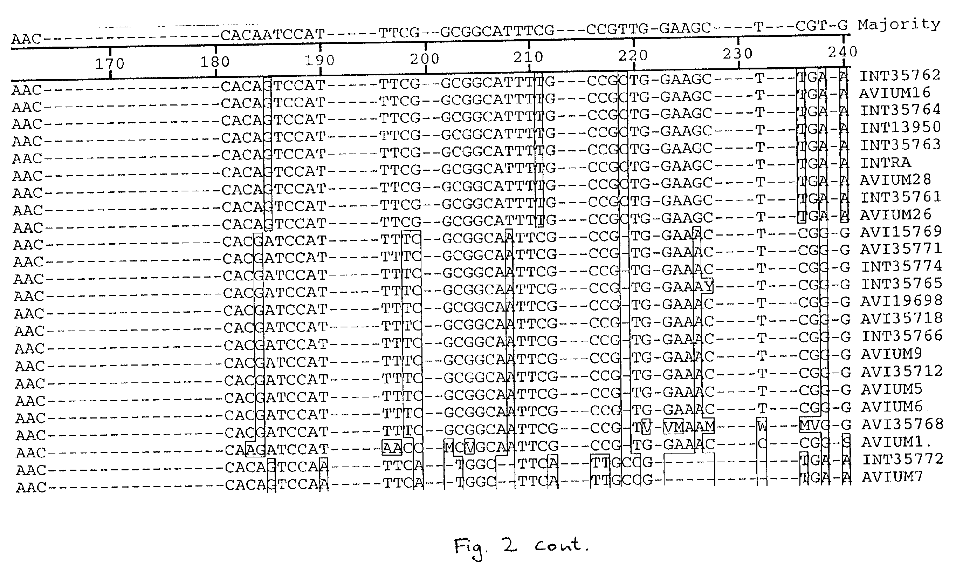

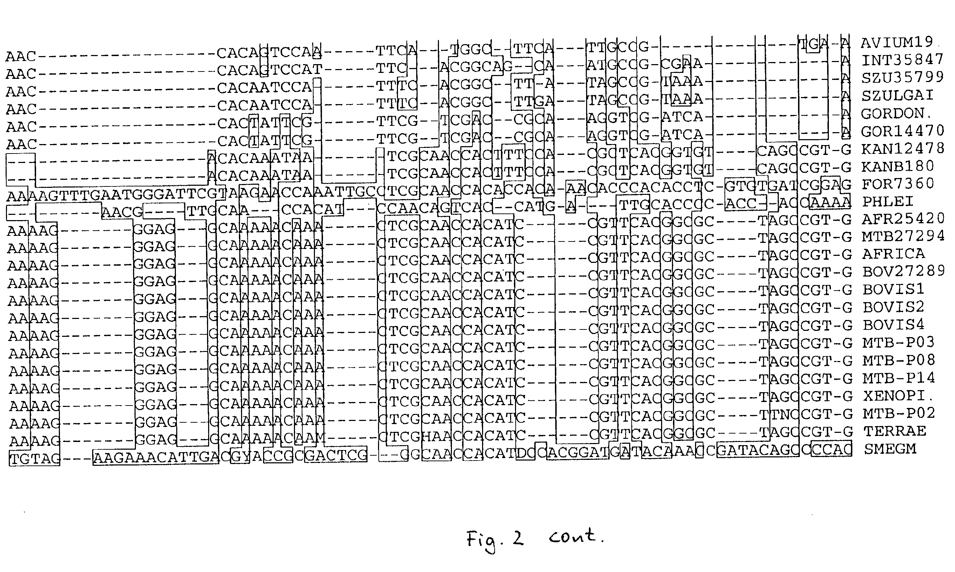

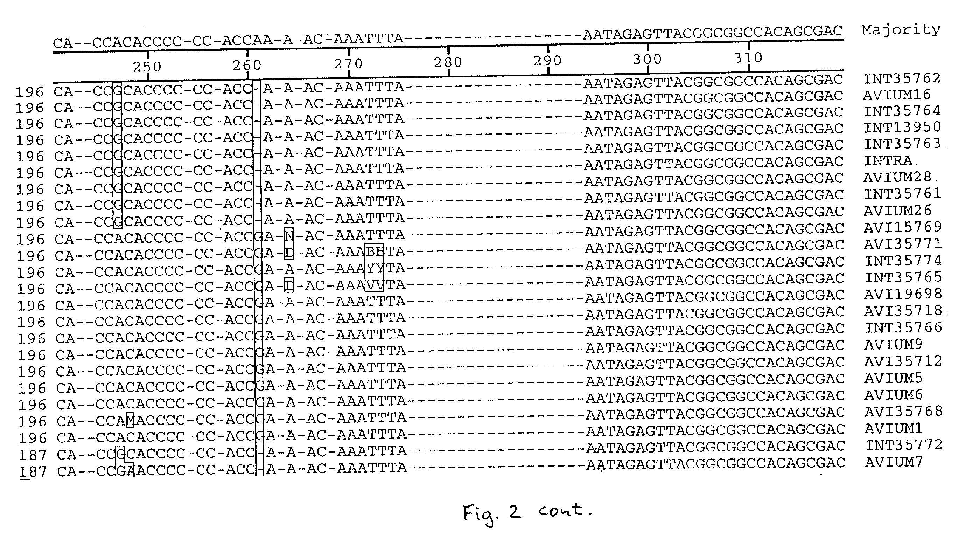

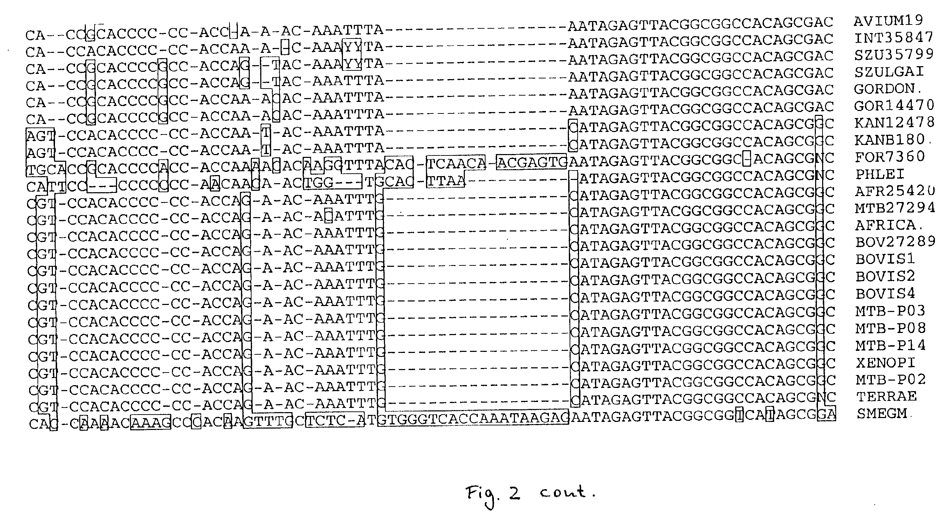

**Figur 2:** Alignment der erfindungsgemäßen Sequenzen (Alignment report of PATENT1.meg). Die 23S/5S-Spacerregion (die Mehrheitssequenz ist in SEQ.ID.NO.4 dargestellt) beginnt mit der Position 140 und endet mit der Position 294 (entsprechend der Zählung der SEQ.ID.NO.1 als Consensus-Sequenz der untersuchten Mykobakterien-Isolate). Das Alignment wurde nach der Clustal Methode mit weighted residue weight table erstellt.

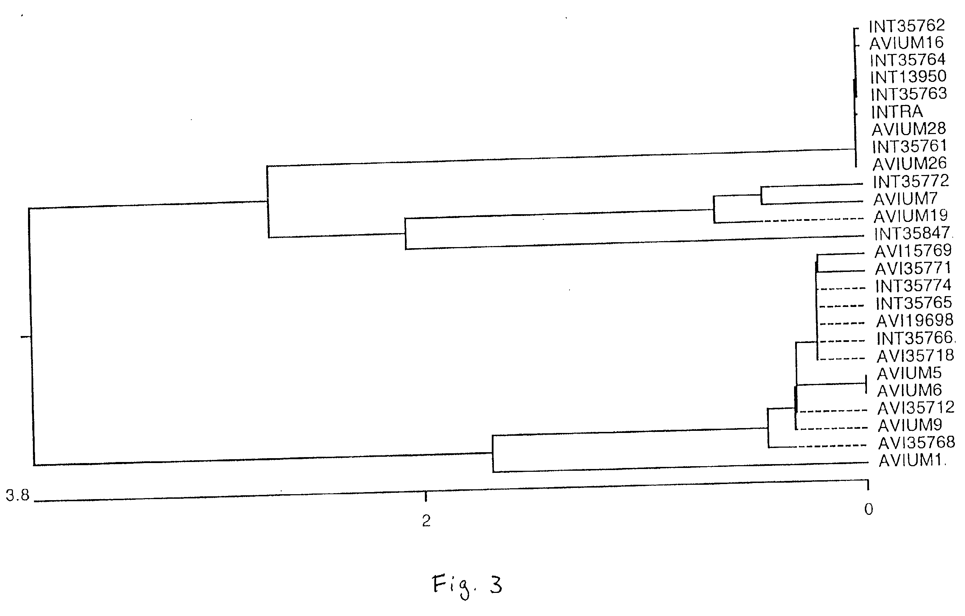

**Figur 3:** Phylogenetischer Stammbaum von untersuchten Isolaten aus der Gruppe des MAI-Komplex (Phylogenetic tree of MAI all.MEG).

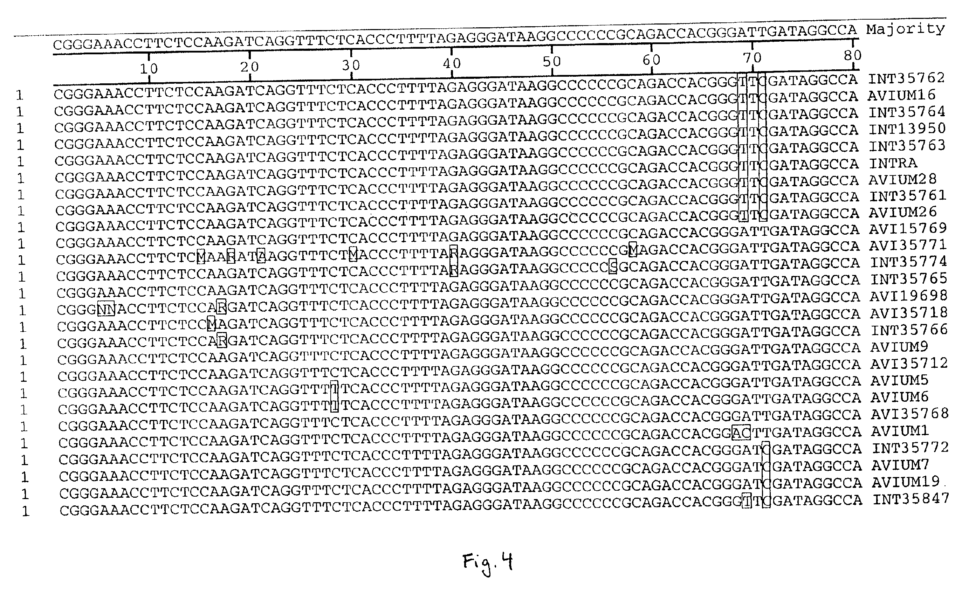

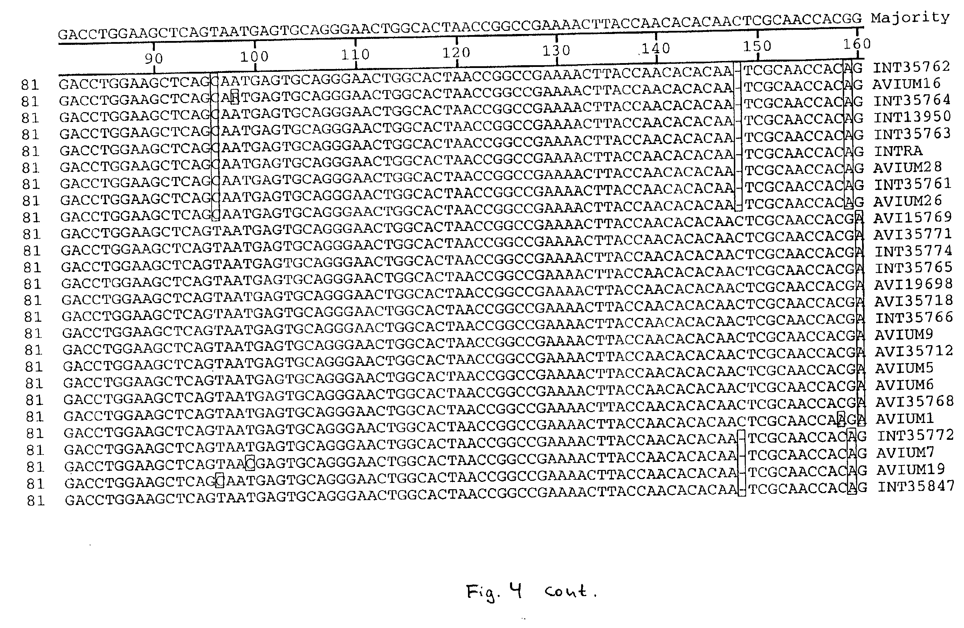

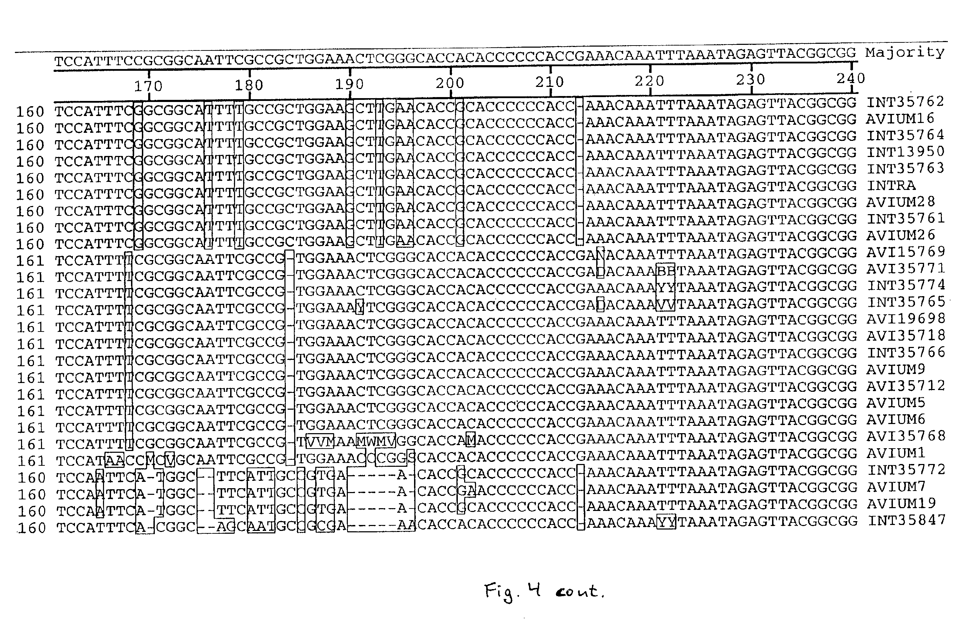

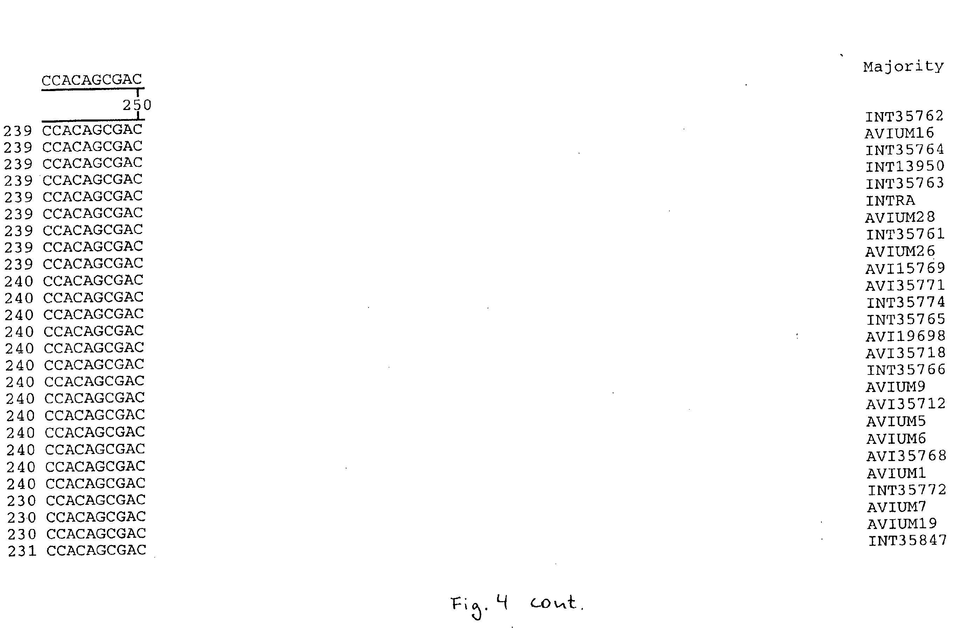

**Figur 4:** Alignment der erfindungsgemäßen Sequenzen von untersuchten Isolaten aus der Gruppe des MAI-Komplex (Aligment Report for MAI all. MEG). Die 23S/5S-Spacerregion der untersuchten Isolaten aus der Gruppe des MAI-Komplex beginnt mit der Position 134 und endet mit der Position 225 (entsprechend der Zählung der SEQ.ID.NO.72). Das Alignment wurde nach der Clustal Methode mit weighted residue weight table erstellt.

Bezüglich der Spezifität der Amplifikation ist festzuhalten, daß im Rahmen der vorliegenden Erfindung nicht ausgeschlossen ist, daß, selbst bei Vorliegen der Spezies, dessen Nukleinsäuren Gegenstand der Amplifikation sind, unspezifische Amplifikate gebildet werden, die aber üblicherweise eine sehr viel größere Länge haben. Während die gewünschten Amplifikate unter Verwendung der beschriebenen Primer MCON1 und MCON2c eine Länge von weniger als 307bp haben und auch bei Amplifikation bei Auswahl entsprechender Sequenzen der Primer für andere Gattungen, Gruppen, Spezies und Subspezies hätten, sind die unspezifischen Amplifikate größer als 400bp. Spezifität im Sinne der Erfindung soll nicht bedeuten, daß diese Artefakte nicht gebildet werden können, selbst wenn dies, abhängig von den sonstigen Amplifikationsbedingungen, relativ selten vorkommen wird.

Die Nukleinsäuresonden der vorliegenden Erfindung sind mindestens 10 Basen lang, besonders bevorzugt zwischen 15 und 40 Basen. Es handelt sich somit um Nukleinsäuren, welche auf einfache Weise durch chemische Synthese in sogenannten (Nukleinsäure-) Synthesizern hergestellt werden können.

Die Gruppen-, Spezies- oder Subspezies-Spezifität wird in der Regel eine Abweichung in 1 oder 2 Basen in Abhängigkeit von den Hybridisierungsbedingungen in den meisten Fällen kein Abbruch getan, sofern die Abweichungen in einer Position geschehen, die keine Bevorzugung einer speziellen Gruppe, Spezies oder Subspezies bedeutet. Es ist selbstverständlich, daß die Anzahl der Abweichungen mit zunehmender Größe der Nukleinsäuresonde zunehmen kann. Erfindungsgemäß sind daher jeweils Sonden bevorzugt, die zu mindestens 90 % homolog oder zu mindestens 90 % komplementär zu einem Teil der oben genannten SEQ.ID.NO. sind. Besonders bevorzugt sind Sonden, welche eine mindestens 12 Basen lange Sequenz (in direkter Aufeinanderfolge) enthalten, die zu 100 % homolog oder 100 % komplementär zu einem Teil dieser SEQ.ID.NO. ist.

Die Gruppen-, Spezies- bzw. Subspezies-Spezifität könnte leiden, wenn die Sonde weitere Gruppen-, Mycobakterienspezies- oder Mycobakteriensubspezies-spezifische Sequenzen oder nicht-Mycobacterium spezifische Sequenzen enthält. Daher sind weitere Mycobakterien-spezifische Sequenzen, sofern sie überhaupt vorliegen, nicht mehr als 15 Basen lang.

Des weiteren kann eine Nukleinsäuresonde im Mycobakterien-unspezifischen Teil funktionelle Nukleotidsequenzen, wie sie beispielsweise für Promotoren oder Origins of Replication (ori) charakteristisch sind, enthalten.

Die erfindungsgemäßen Amplifikationsverfahren verwenden bevorzugt 2 oder mehr Primer, von denen einer zu einem ersten Strang der zu amplifizierenden Sequenz und der andere zum dazu komplementären Strang der nachzuweisenden Sequenz komplementär ist.

Ein bevorzugtes Amplifikationsverfahren wird dadurch realisiert, daß der erste Primer mit der zu amplifizierenden Nukleinsäure hybridisiert und mit Mononukleosidtriphosphaten unter Einwirkung einer DNS-Polymerase unter Verwendung des ersten Strangs der zu amplifizierenden Nukleinsäure als Templat verlängert wird, das Verlängerungsprodukt von der Templatnukleinsäure getrennt und der obige Vorgang unter Verwendung des zweiten Primers und dem Verlängerungsprodukt als Templat wiederholt wird. In jedem Zyklus werden daher ab dem zweiten Zyklus pro Nukleinsäure zwei Verlängerungsprodukte gebildet. Es handelt sich somit um ein theoretisch exponentielles Amplifikationsverfahren. Für Details wird auf EP-A-0 201 184 verwiesen.

Auch bei den auf Transkriptionsreaktionen beruhenden Amplifikationsverfahren werden im allgemeinen Fall 2 Nukleinsäuresonden eingesetzt, die entweder auf gegenläufigen Strängen oder auf einem Strang der zu amplifizierenden Nukleinsäure hybridisiert werden können.

Erfindungsgemäß handelt es sich für das gattungsspezifische Amplifikationsverfahren bei allen als Primer fungierenden Nukleinsäuresonden um Mycobakterien-gattungsspezifische Sonden. In einem besonders bevorzugten Fall hybridisiert einer der Primer mit einem Strang der Mycobakterien-Nukleinsäure in der 23S-rDNS-Region und der andere mit dem Gegenstrang in der 5S-rDNS-Region. Dadurch wird erreicht, daß das amplifizierte Teilstück der Nukleinsäuresequenz der Mycobakterien sowohl Teile der 23S-rDNS-Region, der 5S-rDNS-Region als auch der dazwischenliegenden Spacerregion umfaßt. Zum Verständnis muß an dieser Stelle darauf hingewiesen werden, daß sich die Amplifikate unterschiedlicher Spezies in der Sequenz unterscheiden, die zwischen den einander zugewandten Enden der ursprünglichen Primer liegt. Dabei handelt es sich eben bevorzugt um Sequenzen der Spacer-Region.

Das erfindungsgemäße gattungsspezifische Amplifikationsverfahren kann für mehrere Zwecke benutz werden. In einer ersten Möglichkeit (z. B. im Sinne eines Screenings) kann die Summe aller in der Probe vorliegenden Spezies der Gattung Mycobakterien nachgewiesen werden. Dies ist beispielsweise möglich, wenn die in der gattungsspezifischen Amplifikation erzeugten Amplifikate mit einer weiteren gattungsspezifischen (gewünschtenfalls nachweisbar markierten) Nukleinsäuresonde zur Hybridisierung gebracht und die gebildeten Hybride nachgewiesen werden. Dabei kann die Nukleinsäuresonde (Nachweissonde) so gewählt werden, daß sie in den Bereichen nahe den ursprünglichen Primerhybridisierungsstellen hybridisiert. Besonders bevorzugt hybridisiert die gattungsspezifische Nachweissonde zwischen den Positionen 109 und 141 sowie 245 und 298 der SEQ.ID.NO.1 bzw. zwischen den Positionen 106 und 172 sowie 214 und 245 der SEQ.ID.NO.5. und ist zwischen 25 und 35 Nukleotide, besonders bevorzugt 26 bis 30 Nukleotide lang. Mit der Veränderung der Länge der Sonde muß die Hybridisierungstemperatur entsprechend angepaßt werden.

Prinzipiell sind für den Nachweis der Amplifikate alle bekannten Nachweisformate verwendbar, beispielsweise Auftrennung nach Größe der Amplifikate (z. B. Gelelektrophorese), aber auch die Blot-Verfahren. Beispielsweise kann schon aufgrund der Größenvariabilitäten der Spacerregion eine Differenzierung von Spezies im einfachen, hochauflösenden Agarosegel durchgeführt werden, was im Sinne eines Screeningverfahrens eine rasche orientierende Information liefert. Auf einen Nachweis, der auf einem Hybridisierungsschritt mit einer Nachweissonde beruht, kann jedoch meist nicht verzichtet werden. Als besonders vorteilhaft hat sich die Verwendung des Reverse-Dot-Blot-Formats erwiesen. Hierzu wird beispielsweise eine gattungsspezifische Nukleinsäuresonde auf einer Membran immobilisiert und anschließend das Produkt der Amplifikationsreaktion auf die immobilisierten Sonden gegeben. Dazu müssen die Amplifikate gegebenenfalls vorher einzelsträngig gemacht werden. Wenn während der Amplifikation markierte Mononukleosidtriphosphate eingebaut wurden, ist der Nachweis der über die Nukleinsäuresonde immobilisierten Amplifikate nach Abwaschen nicht gebundener Nukleinsäuren auf einfache Weise möglich. Ein solches Format ist beispielsweise in der EP-A-0 237 362 beschrieben. Auf den Inhalt dieser Patentanmeldung wird hiermit vollinhaltlich Bezug genommen.

Ein Nachweis ist jedoch auch über die sogenannten Sandwich-Verfahren möglich, bei denen neben der immobilisierten Nukleinsäuresonde eine weitere, markierte gattungsspezifische Nukleinsäuresonde (Nachweissonde) eingesetzt wird, die mit den Amplifikaten an einer anderen Position hybridisiert als die Festphasen-gebundene Sonde. Dieses Verfahren ist prinzipiell in EP-B-0 079 139 beschrieben.

Das erfindungsgemäße gattungsspezifische Amplifikationsverfahren ermöglicht jedoch auch den verläßlichen und unkomplizierten spezifischen Nachweis von Mycobakterien-Spezies oder -Subspezies (z. B. für den klinischen Routinealltag) aber auch Gemischen von Spezies, bevorzugt pathologisch differenzierbaren Komplexen (z. B. eine Unterscheidung von MTB-Komplex von MAI-Komplex). Für diesen Fall kann im Anschluß an das gattungsspezifische Amplifikationsverfahren eine Hybridisierung mit einer (gewünschtenfalls markierten) Sonde mit der gewünschten Spezifität (Nachweissonde) durchgeführt werden, die im amplifizierten Bereich zwischen den aufeinander zu zeigenden Enden der Primer hybridisiert. In SEQ.ID-Nos. 5 bis 55 sind Sequenzen, aus welchen die speziesspezifischen Sequenzen ausgewählt werden können, für einzelne Subspezies angegeben. Die Länge der Amplifikate ergibt sich aus den Hybridisierungspositionen der Primer.

Das erfindungsgemäße Verfahren ermöglicht den multiplen Nachweis von Mycobakterien-Spezies unter Verwendung einer einzigen in einer vorgeschalteten gattungsspezifischen Amplifikationsreaktion hergestellten Reaktionsmischung, die Amplifikate von Teilen der ein der Probe anwesenden Mycobakterien-Spezies enthält. Der Nachweis der Amplifikationsprodukte kann dann mit Hilfe von Sonden gewünschter Spezifität, auch eines Gemisches von Sonden geschehen. Ein Vorteil der Erfindung ist daher, daß sie einen einfacheren, d. h. weniger komplexen Nachweis von Bakterien der Gattung Mycobacterium ermöglicht.

Eine ebenfalls sehr bevorzugte Ausführungsform des erfindungsgemäßen Verfahrens ist gekennzeichnet durch eine oder mehrere Mycobakterien-gruppenspezifische Amplifikation (z. B. mit MTB-gruppenspezifischen Primern und in einem getrennten Ansatz mit MAIgruppenspezifischen Primern) und Nachweis, ob die Probe für eine oder mehrere dieser Gruppen und für welche Gruppe ein positives Ergebnis liefert, und anschließende speziesoder subspeziesspezifische Amplifikation mit Bestimmung, welche der Spezies dieser Gruppe tatsächlich in der Probe enthalten ist (z. b. M. tuberculosis-spezifischen Primern und Sonden). Dies kann zu einer erheblichen Senkung der Kosten für eine Suche nach dem Erreger führen. Oft ist auch schon die Zugehörigkeit zu einer bestimmten Gruppe ausreichend für die Entscheidung über die Notwendigkeit und Art der medikamentösen Behandlung, welche für die unterschiedlichen Gruppen unterschiedlich sein kann.

Eine weitere bevorzugte Ausführungsform des erfindungsgemäßen Verfahrens verwendet Primer einer unterschiedlichen Spezifität zur Amplifikation. So ist es beispielsweise möglich, einen Primer gattungsspezifisch zu wählen, den anderen jedoch gruppen-, spezies- oder subspeziesspezifisch. In diesem Fall wird die Amplifikation der Spezifität des zweiten Primers folgen. Auch bei Verwendung eines zweiten gruppenspezifischen Primers ist beispielsweise ein gruppenspezifischer, speziesspezifischer oder subspeziesspezifischer Nachweis mit Hilfe einer entsprechenden Nachweissonde möglich.

Ebenfalls Gegenstand der Erfindung ist ein Paar von Primern, bevorzugt gruppenspezifische, zur Amplifikation von Mycobakterien-Nukleinsäuren, von denen einer mit einem Strang der 23S-Region und der andere mit dem Gegenstrang in der 5S-Region des Mycobacterium-Genoms hybridisiert.

Dadurch, daß im Zuge der Erfindung herausgefunden wurde, daß die 23S/5S-Spacerregion bei Mycobakterien die Differenzierung zwischen einzelnen Gruppen, Spezies und sogar Subspezies erlaubt, eröffnet sich auch die Möglichkeit einer gruppen-, spezies- oder subspezies-spezifischen Amplifikation mit anschließendem Nachweis, der wiederum um eine Stufe spezifischer sein kann. Sofern eine gruppenspezifische Amplifikation vorgenommen wurde, ist somit ein gruppenspezifischer, spezies-spezifischer oder subspezies-spezifischer Nachweis möglich. Mindestens einer der Primer, bevorzugt jedoch beide Primer, werden für diese Fälle aus der 23S/5S-Spacerregion gewählt, besonders bevorzugt natürlich aus der Region, die die geforderte Spezifität gewährleistet.

Ebenfalls Gegenstand der Erfindung ist eine besonders vorteilhafte Diagnosestrategie unter Verwendung einer Kombination der erfindungsgemäßen Nach-Amplifikations- und Nachweisverfahren. Die erfindungsgemäßen Verfahren ermöglichen nämlich eine sehr flexible Antwort auf therapeutische Fragestellungen und die weitere therapeutische Behandlung: der Kliniker ist es gewohnt, symptomorientiert nach seiner Diagnostik zu fragen; die Zusammenstellung der Fragestellungen klinisch tätiger Ärzte zeigt, daß nur in etwa 5% gezielt nach einem Erreger gefragt wird und in den verbleibenden 95% nach einem beliebig zu findenden Erreger. Der Diagnostiker, der nun eine derartige Probe mittels der PCR bearbeiten will, kann entweder nun ein groß angelegtes Panel an verschiedenen PCR-Systemen anbieten - in der Hoffnung, daß er das z.B. vorhandene Mycobacterium kansasii derart findet - und wird unter Umständen nur sagen können, was er alles nicht gerunden hat, oder er wird die Probe nach aufwendigen konventionellen Kulturbedingungen behandeln und nach entsprechender kultureller und biochemischer Charakterisierung auf seiner Platte sehen, daß Mycobacterium kansasii nachgewiesen werden kann. Die verschiedenen Amplifikationsverfahren, wie z.B. die PCR werden derzeit in der klinischen Routinediagnostik derart gehandhabt, daß sie allgemein zu 90% zum Ausschluß, nicht aber zur positiven Diagnostik benutzt werden können. Evident wird dieses Problem z.B. bei der klinisch unscharfen aber wichtigen Fragestellungen wie "Sepsis?" oder "Entzündung" wo auch für einen guten Kliniker meist keine oder nur wenige Anhaltspunkte hinsichtlich des in Frage kommenden Keimes vorliegen. Neben den wenigen Einzelfällen, in denen nach einer spezifischen PCR-Diagnostik gefragt wird, muß die PCR für die Routinediagnostik versuchen, die klassische klinische Mikrobiologie in ihrem Entscheidungsalgorithmus zu imitieren. Die PCR muß in einem Art Screening-Verfahren (Mycobakterien ja/nein?, Mycobacterium avium intracellulare ja/nein?) die jeweilige Probe testen, ob überhaupt z.B. mycobakterielle DNA oder RNA vorliegt und im positiven Fall erfolgt eine weitere Differenzierung aus dem gleichen Probenansatz heraus, oder diese Differenzierung geschieht in parallelen Formaten (z.B. Chip-Systeme). Dieses Vorgehen ermöglicht es auch - unter ökonomischen Aspekten - der Tatsache Rechnung zu tragen, daß ca. 60-90% aller mykobakteriellen Proben in der derzeit durchgeführten klassischen Diagnostik (Kultur, Serologie) keinen Anhalt für eine Mykobakterien-Infektion ergaben; nicht weil die Diagnostik schlecht ist, sondern weil das klinische Raster schlecht ist bzw. die Symptome diffus sind.

Ebenfalls Gegenstand der Erfindung ist ein Paar von Primern zur Amplifikation von Nukleinsäuren einer speziellen Gruppe, einer Spezies oder einer Subspezies von Mycobakterien, von denen mindestens einer mit einem Strang der 23S/5S-Spacerregion und der andere mit dem Gegenstrang, bevorzugt ebenfalls innerhalb der Spacerregion, hybridisiert.

Ebenfalls Gegenstand der Erfindung ist eine doppelsträngige Mycobakterien-spezifische Nukleinsäure, enthaltend einen Teil der Spacerregion zwischen 5S-rDNS und 23S-rDNS mit einer Länge von zwischen 50 und 200, bevorzugt 50 und 120 Nukleotiden. Bevorzugt ist diese doppelsträngige Nukleinsäure höchstens 307 bp lang und ist das Produkt einer gruppen-, spezies- oder subspeziesspezifischen Amplifikationsreaktion.

Ebenfalls Gegenstand der Erfindung ist ein Reagenzkit zum Nachweis von Mycobakterien-Gattung-, Gruppen, -Spezies oder -Subspezies enthaltend mindestens einen Mycobakterien-gattungsspezifische Primer und mindestens eine Mycobakterium-gattungs-, gruppen-, -spezies- oder -subspezies-spezifische Sonde.

In Figur 1 ist der phylogenetische Stammbaum einiger Mycobakterien gezeigt (Phylogenetic tree of PATENT1.meg).

In Figur 2 ist ein Alignment der erfindungsgemäßen Sequenzen gegeben (Alignment report of PATENT1.meg). Die 23S/5S-Spacerregion beginnt mit der Position 140 und endet mit der Position 294 (entsprechend der Zählung der SEQ.ID.NO.1 als Consensus-Sequenz der untersuchten Mykobakterien). Das Alignment wurde nach der Clustal Methode mit weighted residue weight table erstellt. Die Herstellung der Alignments erfolgte durch eine multiple alignment procedure unter Verwendung der Clustal Methode. Zur Herstellung des vorliegenden Alignments und der hieraus abgeleiteten Phylogenie und den Mehrheitssequenzen wurden eine gap penalty und eine gap length penalty von jeweils 10 zugrunde gelegt.

In Figur 3 ist der phylogenetische Stammbaum von untersuchten Isolaten aus der Gruppe des MAI-Komplex (Phylogenetic tree of MAI all.MEG). In Figur 4 ist ein Alignment der erfindungsgemäßen Sequenzen von untersuchten Isolaten aus der Gruppe des MAI-Komplex gegeben (Aligment Report for MAI all. MEG). Das Alignment wurde nach der Clustal Methode mit weighted residue weight table erstellt. Die Herstellung der Alignments erfolgte durch eine multiple alignment procedure unter Verwendung der Clustal Methode. Zur Herstellung des vorliegenden Alignments und der hieraus abgeleiteten Phylogenie und den Mehrheitssequenzen wurden eine gap penalty und eine gap length penalty von jeweils 10 zugrunde gelegt.

Die folgenden Beispiele sollen die Erfindung näher erläutern:

### Beispiel 1: Bakteriengewinnung und DNA-Aufschluß

Die untersuchten Mykobakterienspecies standen als Flüssigkultur zur Verfügung; die Präparation erfolgte jeweils aus der inaktivierten Keimsuspension. Die Kolonien, wurden zuerst für 10 Minuten bei 15.000xg zentrifugiert, zweimal mit 0,9% NaCl gewaschen und anschließend ebenfalls in 1ml H₂O resuspendiert. 1 ml der inaktivierten Mykobakteriensuspension wurden mit 500µl Lysozym für 90 Minuten bei 37°C inkubiert. Dann wurden 400µl Proteinase K und 100µl Natrium-Dodecyl-Sulfat 20% hinzugegeben und für weitere 12-15 Stunden bei 55°C belassen. Das Lysat wurde mit 2ml Phenol/Chloroform/ Isoamylalkohol (25:24:1) versetzt und unter Schütteln homogenisiert, die Proben für 5 Minuten auf Eis inkubiert und danach 10 Minuten bei 4°C mit 10.000g zentrifugiert. Der wäßrige Überstand wurde abgehoben und mit gleichem Volumen Chloroform/Isoamylalkohol (24:1) versetzt, durch Schütteln homogenisiert und wiederum 5 Minuten auf Eis inkubiert. Nach Zentrifugation für 10 Minuten bei 4° C und 10.000g wurde der wäßrige Überstand in ein steriles Röhrchen überführt.

Dem wäßrigen Überstand wurden 2 Vol Ethanol und 1/10Vol 3M Natriumazetat (pH 5,3) zugegeben und die DNS zum Ausfällen für 12 Stunden bei -20°C gelagert. Es folgte eine Zentrifugation bei 4° C für 15 Minuten und 15.000g an mit anschließendem zweimaligem Waschen des DNS-Pellets mit 80% Ethanol. Das gewaschenen Bakterienpellet wurde luftgetrocknet und in 150µl TE-Puffer resuspendiert. Die so gewonnenen Nukleinsäuren konnten in den Amplifikations - und Hybridisierungsexperimenten eingesetzt werden.

### Beispiel 2: Nachweis von Mykobakterien (Gattung, Spezies und Subspezies)

Der Nachweis von Mykobakterien geschah durch erfindungsgemäße Amplifikation unter Einbau von mit Digoxigenin markierten Primern.

Beispielhaft werden für die spezifische Amplifikation der Gattung Mykobakterien, Gruppen und Spezies bzw. Subspezies folgende Primer dargestellt:

| **Spezifisch zu amplifizierendes Target** | **Primer** | **Größe des PCR-Produktes** |
|---|---|---|
| Mycobakterien | MCONl/MCON2c | ca. 239 - ca. 307bp |
| M. tuberculosis complex = MTB-Komplex | MTC1/MCON2c oder | ca. 146bp |
| | MTC2C/MCON1 | ca. 177bp |
| M. avium intracellulare Komplex = MAI-Komplex | MAI-1c/MCON1 | ca. 303bp |
| M. intrazellulare (ATCC 35772) | INT35772/MCON1 | 177bp |

### (A) Nachweisverfahren mit gattungsspezifischer Mycobacteriaceae Amplifikation und anschließender Sondendifferenzierung und spezifische Sonden für den Nachweis des MTB-Komplex

Die Mykobakterien einer Probe wurden mittels PCR unter Verwendung gattungsspezifischer Primer amplifiziert und anschließend mit Nachweissonden unterschiedlicher Spezifität nachgewiesen. Als gattungsspezifische Primer wurden Primer verwendet, die üblicherweise außerhalb der Spacerregion hybrisieren. Primer: MCON1 und MCON2c

Die 25µl PCR wurde auf einem Thermocycler von Perkin Elmer (PE 9600) unter folgenden Bedingungen durchgeführt:

| **Reagenz** | **Konz. Stammlösungen** | **Menge in µl** |
|---|---|---|
| H2O | | 9,85 |
| 10 x PCR-Puffer | | 2,5 |
| MgCl2 | 25mM | 1,0 |
| DMSO | 100% | 1,25 |
| DNTPs | 200mM | 1,0 |
| Taq-Polymerase | 5U/µl | 0,4 |
| Primer | 10µmol | je 4,0 |
| DNA | 250ng/µl | je 1 |
| Thermoprofil: 1. 94°C, 5min, 2. 94°C, 20 sec, 3. 47°C, 72°C, 4. 72°C, 20 sec, 5. 72°C, 10min, 6. 4°C. Zyklenzahl: (Schritt 2.-4.): 40x | | |

| **MCON1/MCON2-amplifizierte Mykobakterien:** | | |
|---|---|---|
| **Detektionstarget** | | **Detektionssonde** |
| Gattung: | Mykobakterien | MCONpro1 oder MCONpro2 |
| Gruppe: | MTB-Komplex | MTCpro1 |
| Spezies: | M. intrazell. ATCC 35772 | INT35772pro1 |

Die Hybridisierung der resultierenden Fragmente mit den spezifischen Sonden erfolgte unter üblichen Bedingungen (z.B. Temperatur 58°C, 60min, Sondenkonzentration : 50ng/ml Hybridisierungslösung, wie im Beipackzettel des DNA-Detection-Kits (Boehringer-Mannheim GmbH, Best.-Nr. 1536111) beschrieben, durchgeführt. Die Sonden unterscheiden sich in ihrer Spezifität. Das Ergebnis der Hybridisierung mit den Sonden MCONpro2 und MTCpro1 ist in Tabelle 2 dargestellt. Keine der mittels MCON1/MCON2c amplifizierten, in Tabelle 2 aufgeführten Bakterien konnte nicht mit MCONpro2 detektiert werden; kein Mitglied des MTB-Komplexes ließ sich nicht mittels MTCpro1 nachweisen.

### (B) MTB-gruppenspezifische Amplifikation mit anschließender spezifischer Hybridisierung

Die Mycobakterien einer Probe wurden mittels PCR unter Verwendung eines gattungsund eines gruppenspezifischen (MTB-Komplex, Mycobacterium tuberculosis Komplex) Primers amplifiziert und anschließend mit Nachweissonden unterschiedlicher Spezifität nachgewiesen. Als gattungsspezifischer Primer diente in dem einen Ansatz MCON2c, der außerhalb der Spacerregion hybridisiert, im anderen Ansatz MCON1. Die MTB-Komplex-spezifischen Primer hybridisieren innerhalb der Spacerregion.

Die PCR-Bedingungen für MTC1/MCON2c-Amplfikation waren wie in Beispiel A). Das Thermoprofil war folgendes:
1. 94°C, 5min; 2. 94°C, 20sec, 3. 53°C, 20sec; 4. 72°C, 20sec; 5. 72°C, 10min; 6. 4°C. Zyklenzahl (Schritt 2.-4.): 40 x

Analog wurde mit MCON1 und einem anderen gruppenspezifischen Primer (MTC2c) amplifiziert. Die PCR-Bedingungen waren analog zu Beispiel A). Das Thermoprofil war folgendes:
1. 94°C, 5min; 2. 94°C, 20sec, 3. 51°C, 20sec; 4. 72°C, 20sec; 5. 72°C, 10min; 6. 4°C. Zyklenzahl (Schritt 2.-4.): 40 x

Anschließend wurden die Amplifikate durch Hybridisierung mit unteschiedlich spezifischen Sonden nachgewiesen. Die Hybridisierungsbedingungen waren wie oben, jedoch die Temperatur 74°C, 60min (für den MTC1-Ansatz), bzw. 58°C, 60min (für den MTC2c- Ansatz).

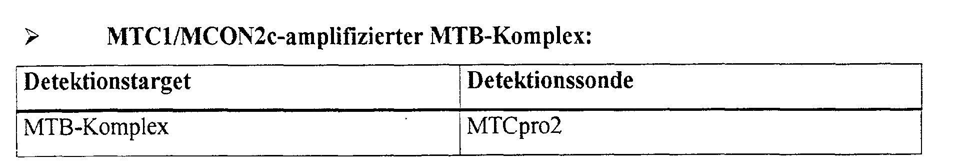

Folgende Mykobakterien wurden mit Hilfe dieser Versuche spezifisch positiv nachgewiesen: Mtb. RMP sensibel, Mtb. RMP resistent, M. bovis 6594/96, M. bovis 6935/96, Mtb. H37 Rv, M. tuberculosis ATCC 27294, M. africanum ATCC 25420.

Folgende Mykobakterien wurden mit Hilfe dieser Versuche spezifisch negativ nachgewiesen:
M. fortuitum 3891/96, M. fortuitum 6646/96, M. gordonae, M. terrae, M. intrazellulare, M. szulgei, M. phlei, M. kansasii, M. xenopi, M. avium ATCC 15769, A. avium ATCC 35718, M. avium ATCC 35771, M. intrazellulare ATCC 35766, M. intrazellulare ATCC 35762, M. intrazellulare ATCC 35765, M. intrazellulare ATCC 35766, M. intrazellulare ATCC 35772, M. intrazellulare ATCC 35774, M. fortuitum ATCC 6841, M. gordonae ATCC 14470, M. kansasii ATCC 12478, M. phlei ATCC 11758, M. szulgei ATCC 35799.

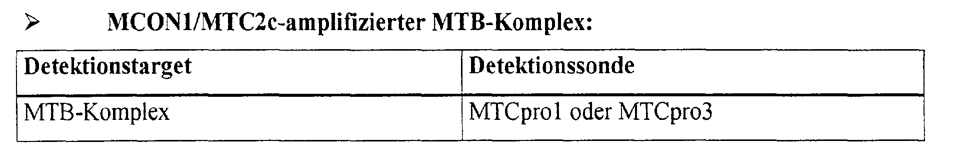

Folgende Mykobakterien wurden mit Hilfe dieser Versuche spezifisch positiv nachgewiesen: Mtb. RMP sensibel, Mtb. RMP resistent, M. bovis 6594/96, M. bovis 6935/96, Mtb. H37 Rv, M. tuberculosis ATCC 27294, M. africanum ATCC 25420 Folgende Mykobakterien wurden mit Hilf dieser Versuche spezifisch negativ nachgewiesen: M. fortuium 3891/96, M. fortuium 6646/96, M. fortuium ATCC 6841, M. gordonae.

### (C) MAI-Gruppenspezifische Amplifikation mit anschließender spezifischer Hybridisierung

| **MCON1/MAI1c/MAI2c-amplifizierter MAI-Komplex:** | |
|---|---|
| **Detektionstarget** | **Detektionssonde** |
| MAI-Komplex | MAIpro1 und MAIpro2 |

Die Mykobakterien einer Probe wurden mittels PCR unter Verwendung eines gattungsund eines oder mehrerer gruppenspezifischer (MAI, Mycobacterium avium intracellulare Komplex) Primer amplifiziert und anschließend mit Nachweissonden unterschiedlicher Spezifität nachgewiesen. Als gattungsspezifischer Primer diente MCON1 aus Beispiel A), der außerhalb der Spacerregion hybridisiert. Die MAI-spezifischen Primer MAI1c und MAI2c hybridisieren innerhalb der Spacerregion. MAI1c ist in der Lage, die sog. T-Isolate des MAI-Komplexe ("T" an Position 178 der Isolate INT35762, AVIUM16, INT35764, INT13590, INT35763, INTRA, AVIUM28, INT35761, AVIUM26) zu amplifizieren, MAI2c die C-Isolate ("C" an Position 179 der Isolate AVI15769, AVI35771, INT35774, INT35765, AVI19698, AVI35766, AVIUM9, AVI35712, AVIUM5, AVIUM6, AVI35768, AVIUM1)

Die PCR-Bedingungen waren wie in Beispiel A). Das Thermoprofil war folgendes: 1. 94°C, 5min; 2. 94°C, 20sec, 3. 53°C, 20sec; 4. 72°C, 20sec; 5. 72°C, 10min; 6. 4°C. Zyklenzahl (Schritt 2.-4.): 40 x

Anschließend wurden die Amplifikate durch Hybridisierung mit unterschiedlich spezifischen Sonden nachgewiesen. Die Hybridisierungsbedingungen waren wie in Beispiel A). Genutzt wurde ein spezifisches Sondengemisch für den Nachweis des MCON1/MAI1c/MAI2c-amplifizierten MAI-Komplexes bestehend aus MAIpro1 und MAIpro2.

### (D) Subspeziesspezifische Amplifikation mit anschließender spezifischer Hybridisierung

Die Mykobakterien einer Probe wurden mittels PCR unter Verwendung eines gattungsund eines subspeziesspezifischen (M. intrazellulare ATCC 35772, INT35772) Primer amplifiziert und anschließend mit einer subspeziesspezifischen Nachweissonden nachgewiesen. Als gattungsspezifischer Primer diente MCON1 aus Beispiel A), der außerhalb der Spacerregion hybridisiert. Die M. intrazellulare-spezifische Primer hybridisiert innerhalb der Spacerregion.

Die PCR-Bedingungen waren wie in Beispiel A). Das Thermoprofil war folgendes:
1. 94°C, 5min; 2. 94°C, 20sec, 3. 62°C, 20sec; 4. 72°C, 20sec; 5. 72°C, 10min; 6. 4°C. Zyklenzahl (Schritt 2.-4.): 40 x

Anschließend wurden die Amplifikate durch Hybridisierung mit einer subspeziesspezifiscehn Sonde nachgewiesen. Die Hybridisierungsbedingungen waren wie in Beispiel A).

| **MCON1/INT35772-amplifizierte M. intrazellulare ATCC35772-Spezies:** | |
|---|---|
| **Detektionstarget** | **Detektionssonde** |
| M. intrazellulare ATCC 35772 | INT35772prol |

Folgende Mykobakterien wurden als Beispiel einer Spezies-Detektion für die Sonde **INT35772Pro1** getestet:
M.avium ATCC 15769, ATCC 19698, ATCC 35712, ATCC 35714, ATCC 35718, ATCC 35768, ATCC 35771, ATCC 35773; M.avium Serotypi 1, 4, 5, 6, 7, 9, 13, 16, 19, 24, 26, 28;
M.intrazellulare ATCC 13950, ATCC 35761, ATCC 35762, ATCC 35763, ATCC 35764, ATCC 35765, ATCC 35766, ATCC 35772, ATCC 35774, ATCC 35847;

Dabei wurden folgende Mykobakterien als **positiv** getestet:
M.intrazellulare ATCC 35772;
und folgende Mykobakterien als **negativ** getestet:
M.avium ATCC 15769, 19698, 35712, 35714, 35718, 35768, 35771, 35773;
M.avium Serotypi 1, 4, 5, 6, 7, 9, 13, 16, 19, 24, 26, 28;
M.intrazellulare ATCC 13950, 35761, 35762, 35763, 35764, 35765, 35766, 35774, 35847

**Tabelle 2:** Darstellung der Hybridisierungsergebnisse verschiedener mit MCON1/MCON2c amplifizierter Mykobakterien-Isolate bei Verwendung der Sonde MCONpro2 (Detektion aller Mykobakterien) bzw. MTCpro1 (Detektion von Mitgliedern des MTB-Komplex); die mit "+" bezeichneten Isolate haben positive Signale (> Leerwert + 2 x SD) in der Mikrotiter-Platten-Hybridisierung ergeben; Mitglieder des MTB-Komplex sind fett unterlegt.

| **Mykobakterium-Species** | | **MCON Pro 2** | **MTC Pro 1** |
|---|---|---|---|
| **M.tb RMP sensibel** | | + | + |
| **M.tb RMP resistent** | | + | + |
| **M.bovis 6594/96** | | + | + |
| **M.bovis 6935/96** | | + | + |
| M.fortuitum 3891/96 | | + | - |
| M.fortuitum 6646/96 | | + | - |
| M.gordonae | ATCC 14470 | + | - |
| **M.tb H37 RV** | | + | + |
| M.gordonae | | + | - |
| M.terrae | | + | - |
| **M.africanum** | | + | + |
| M.intrazellulare | | + | - |
| M.szulgai | | + | - |
| M.phlei | | + | - |
| M.kansasii | | + | - |
| M.xenopi | | + | - |
| **M.tuberculosis** | ATCC 27294 | + | + |
| M.avium | ATCC 15769 | + | - |
| M.avium | ATCC 35712 | + | - |
| M.avium | ATCC 35718 | + | - |
| M.avium | ATCC 35773 | + | - |
| M.avium | ATCC 35771 | + | - |
| M.intrazellulare | ATCC 35774 | + | - |
| M.intrazellulare | ATCC 35765 | + | - |
| M.intrazellulare | ATCC 35766 | + | - |
| M.intrazellulare | ATCC 35762 | + | - |
| M.intrazellulare | ATCC 35761 | + | - |
| M.intrazellulare | ATCC 35772 | + | - |
| **M.africanum** | **ATCC 25420** | + | + |
| **M.bovis** | **ATCC 27289** | + | + |
| M.fortuitum | ATCC 6841 | + | - |
| M.gordonae | ATCC 14470 | + | - |
| M.kansasii | ATCC 12478 | + | - |
| M.phlei | ATCC 11758 | + | - |
| M.szulgai | ATCC 35799 | + | - |

Mit den Primern MCON1/MCON2 ließen sich bei folgenden bakteriellen Spezies keine PCR-Produkte herstellen: E. coli, Actinobacter jejunii, Pseudomas aeruginosa, Corynebacterium diphtheriae, Serratia marcescens, Proteus mirabilis, Proteus vulgaris, Yersinia enterocolitica, Morganella morganii, Salmonella typhi, Salmonella paratyphi, Streptoccocus (Str.) epidermidis, Str. pneumoniae, Str. pyogenes, Str. Gruppe A, Str. Gruppe B, Bacillus fragilis, Bacillus subtilis, Klebsiella pneumoniae NCTC 8172, Citrobacter freundii, Shigella sonnei, Enterobacter cloacae DSM 30054, Borrelia burgdorferi, Chlamydia pneumoniae.

MYCOBAK.APP
SEQUENZPROTOKOLL

<110> BioChip Technologies GmbH

<120> Nukleinsäuresequenzen und Verfahren zum gattungs-,
      gruppen-, spezies- und subspeziesspezifischen Nachweis
      von Mycobakterien

<130> 135.4

<140> 01110840.4
<141> 2001-05-04

<160> 72

<170> PatentIn Ver. 2.1

<210> 1
<211> 318
<212> DNA
<213> Künstliche Sequenz

<220>
<223> Beschreibung der künstlichen Sequenz:
      Mehrheitssequenz, Position 1-318;

<220>
<221> misc_feature
<222> (1)..(318)
<223> Mehrheitssequenz (Konsensussequenz)

<220>
<221> misc_difference
<222> 57,101,102,112,113,145,151,155,(163)..(179),(190)..(194),
      199,200,(212)..(214),221,(227)..(230),(232)..(234),238,242,
      243,253,256,262,264,267,(275)..(292)
<223> n=a,t,c oder g; n sind keine Bestandteile der
      Mehrheitssequenz

<400> 1
cgggaaacct tctccaagat caggtttctc acccttttag agggataagg ccccccngca 60
gaccacgggt tcgataggcc agacctggaa gctcagtaat nngagtgcag gnngaactgg 120
cactaaccgg ccgaaaactt accanacaca ncaantcgca acnnnnnnnn nnnnnnnnnc 180
acaatccatn nnnnttcgnn acggcatttc gnnnccgtcg ngaagcnnnn tnnncgtngc 240
annccacacc ccnccnacca ananacnaaa tttannnnnn nnnnnnnnnn nnaatagagt 300
tacggcggcc acagcgac                                                318


<210> 2
<211> 138
<212> DNA
<213> Künstliche Sequenz

<220>
<223> Beschreibung der künstlichen Sequenz:
      Mehrheitssequenz der partiellen 3'-Region des
      23S-Genabschnittes, Position 1-138 der SEQ ID NO 1

<220>
<221> misc_feature
<222> (1)..(138)
<223> Mehrheitssequenz (Konsensussequenz) der partiellen
      3'-Region des 23S-Genabschnittes, Position 1-138
      der SEQ ID NO 1

<220>
<221> misc_difference
<222> 57,101,102,112,113

MYCOBAK.APP
<223> n=a,t,c oder g; n sind nicht Bestandteile der
      Mehrheitssequenz

<400> 2
cgggaaacct tctccaagat caggtttctc acccttttag agggataagg ccccccngca 60
gaccacgggt tcgataggcc agacctggaa gctcagtaat nngagtgcag gnngaactgg 120
cactaaccgg ccgaaaac                                               138


<210> 3
<211> 25
<212> DNA
<213> Künstliche Sequenz

<220>
<223> Beschreibung der künstlichen Sequenz:
      Mehrheitssequenz der partiellen 5'-Region des
      5S-Genabschnittes, Position 294-318 der SEQ ID NO
      1

<400> 3
atagagttac ggcggccaca gcgac                                       25


<210> 4
<211> 155
<212> DNA
<213> Künstliche Sequenz

<220>
<223> Beschreibung der künstlichen Sequenz:
      Mehrheitssequenz (Konsensussequenz) der
      23S-5S-Spacerregion, Position 139-294 der SEQ ID
      NO 1

<220>
<221> misc_feature
<222> (1)..(155)
<223> Mehrheitssequenz der 23S-5S-Spacerregion, Position
      139-294 der SEQ ID NO 1

<220>
<221> misc_difference
<222> 7,13,17,(25)..(41),(52)..(56),61,62,(74)..(76),83,
      (89)..(92),(94)..(96),100,104,105,115,118,124,126,
      129,(137)..(154)
<223> n=a,t,c oder g; n sind nicht Bestandteile der
      Mehrheitssequenz

<400> 4
ttaccanaca cancaantcg caacnnnnnn nnnnnnnnnn ncacaatcca tnnnnnttcg 60
nnacggcatt tcgnnnccgt cgngaagcnn nntnnncgtn gcannccaca ccccnccnac 120
caananacna aatttannnn nnnnnnnnnn nnnna                            155


<210> 5
<211> 263
<212> DNA
<213> Mycobacterium tuberculosis ATCC 27294

<400> 5
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aacttacaac accctccctt ttggaaaagg gaggcaaaaa caaactcgca 180
accacatccg ttcacggcgc tagccgtgcg tccacacccc ccaccagaac acatttgcat 240
agagttacgg cggccacagc ggc                                         263

MYCOBAK.APP
<210> 6
<211> 239
<212> DNA
<213> Mycobacterium intracellulare ATCC 35772

<400> 6
cgggaaacct tctccaagat caggtttctc acccttttag agggataagg ccccccgcag 60
accacgggat cgataggcca gacctggaag ctcagtaatg agtgcaggga actggcacta 120
accggccgaa aacttaccaa cacacaatcg caaccacagt ccaattcatg gcttcattgc 180
cgtgaacacc gcacccccca ccaaacaaat ttaaatagag ttacggcggc cacagcgac 239

<210> 7
<211> 263
<212> DNA
<213> Mycobacterium bovis ATCC 27289

<400> 7
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aacttacaac accctccctt ttggaaaagg gaggcaaaaa caaactcgca 180
accacatccg ttcacggcgc tagccgtgcg tccacacccc ccaccagaac aaatttgcat 240
agagttacgg cggccacagc ggc 263

<210> 8
<211> 249
<212> DNA
<213> Mycobacterium avium ATCC 35771

<400> 8
cgggaaacct tctcmaarat aaggtttctm acccttttar agggataagg cccccgmag 60
accacgggat tgataggcca gacctggaag ctcagtaatg agtgcaggga actggcacta 120
accggccgaa aacttaccaa cacacaactc gcaaccacga tccattttcg cggcaattcg 180
ccgtggaaac tcgggcacca caccccccac cgadacaaab btaaatagag ttacggcggc 240
cacagcgac 249

<210> 9
<211> 248
<212> DNA
<213> Mycobacterium intracellulare ATCC 35763

<400> 9
cgggaaacct tctccaagat caggtttctc acccttttag agggataagg ccccccgcag 60
accacgggtt cgataggcca gacctggaag ctcagcaatg agtgcaggga actggcacta 120
accggccgaa aacttaccaa cacacaatcg caaccacagt ccatttcggc ggcattttgc 180
cgctggaagc ttgaacaccg caccccccac caaacaaatt taaatagagt tacggcggcc 240
acagcgac 248

<210> 10
<211> 261
<212> DNA
<213> Mycobacterium africanum ATCC 25420

<400> 10
cgggaaacct tctccaagat caggtttctc acccacttgg tgggataagg ccccgcagaa 60
cacgggttca ataggtcaga cctggaagct cagtaatggg tgtagggaac tggtgctaac 120
cggccgaaaa cttacaacac cctccctttt ggaaaaggga ggcaaaaaca aactcgcaac 180
cacatccgtt cacggcgcta gccgtgcgtc cacacccccc accagaacaa atttgcatag 240
agttacggcg gccacagcgg c 261

<210> 11
<211> 263
<212> DNA
<213> Mycobacterium africanum

MYCOBAK.APP

<400> 11
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aacttacaac accctccctt ttggaaaagg gaggcaaaaa caaactcgca 180
accacatccg ttcacggcgc tagccgtgcg tccacacccc ccaccagaac aaatttgcat 240
agagttacgg cggccacagc ggc 263

<210> 12
<211> 249
<212> DNA
<213> Mycobacterium avium ATCC 15769

<400> 12
cgggaaacct tctccaagat caggtttctc acccttttag agggataagg ccccccgcag 60
accacgggat tgataggcca gacctggaag ctcagtaatg agtgcaggga actggcacta 120
accggccgaa aacttaccaa cacacaactc gcaaccacga tccattttcg cggcaattcg 180
ccgtggaaac tcgggcacca caccccccac cganacaaat ttaaatagag ttacggcggc 240
cacagcgac 249

<210> 13
<211> 249
<212> DNA
<213> Mycobacterium avium ATCC 19698

<400> 13
cgggnnacct tctccargat caggtttctc acccttttag agggataagg ccccccgcag 60
accacgggat tgataggcca gacctggaag ctcagtaatg agtgcaggga actggcacta 120
accggccgaa aacttaccaa cacacaactc gcaaccacga tccattttcg cggcaattcg 180
ccgtggaaac tcgggcacca caccccccac cgaaacaaat ttaaatagag ttacggcggc 240
cacagcgac 249

<210> 14
<211> 249
<212> DNA
<213> Mycobacterium avium ATCC 35712

<400> 14
cgggaaacct tctccaagat caggtttctc acccttttag agggataagg ccccccgcag 60
accacgggat tgataggcca gacctggaag ctcagtaatg agtgcaggga actggcacta 120
accggccgaa aacttaccaa cacacaactc gcaaccacga tccattttcg cggcaattcg 180
ccgtggaaac tcgggcacca caccccccac cgaaacaaat ttaaatagag ttacggcggc 240
cacagcgac 249

<210> 15
<211> 249
<212> DNA
<213> Mycobacterium avium ATCC 35718

<400> 15
cgggaaacct tctccmagat caggtttctc acccttttag agggataagg ccccccgcag 60
accacgggat tgataggcca gacctggaag ctcagtaatg agtgcaggga actggcacta 120
accggccgaa aacttaccaa cacacaactc gcaaccacga tccattttcg cggcaattcg 180
ccgtggaaac tcgggcacca caccccccac cgaaacaaat ttaaatagag ttacggcggc 240
cacagcgac 249

<210> 16
<211> 249
<212> DNA
<213> Mycobacterium avium ATCC 35768

<400> 16
cgggaaacct tctccaagat caggtttctc acccttttag agggataagg ccccccgcag 60

MYCOBAK.APP
accacgggat tgataggcca gacctggaag ctcagtaatg agtgcaggga actggcacta 120
accggccgaa aacttaccaa cacacaactc gcaaccacga tccattttcg cggcaattcg 180
ccgtvvmaam wmvggcacca maccccccac cgaaacaaat ttaaatagag ttacggcggc 240
cacagcgac 249

<210> 17
<211> 249
<212> DNA
<213> Mycobacterium avium Serovar 1

<400> 17
cgggaaacct tctccaagat caggtttctc acccttttag agggataagg ccccccgcag 60
accacggact tgataggcca gacctggaag ctcagtaatg agtgcaggga actggcacta 120
accggccgaa aacttaccaa cacacaactc gcaaccaaga tccataaccm cvgcaattcg 180
ccgtggaaac ccggscacca caccccccac cgaaacaaat ttaaatagag ttacggcggc 240
cacagcgac 249

<210> 18
<211> 248
<212> DNA
<213> Mycobacterium avium Serovar 16

<400> 18
cgggaaacct tctccaagat caggtttctc acccttttag agggataagg ccccccgcag 60
accacgggtt cgataggcca gacctggaag ctcagcartg agtgcaggga actggcacta 120
accggccgaa aacttaccaa cacacaatcg caaccacagt ccatttcggc ggcattttgc 180
cgctggaagc ttgaacaccg caccccccac caaacaaatt taaatagagt tacggcggcc 240
acagcgac 248

<210> 19
<211> 239
<212> DNA
<213> Mycobacterium avium Serovar 19

<400> 19
cgggaaacct tctccaagat caggtttctc acccttttag agggataagg ccccccgcag 60
accacgggat cgataggcca gacctggaag ctcagcaatg agtgcaggga actggcacta 120
accggccgaa aacttaccaa cacacaatcg caaccacagt ccaattcatg gcttcattgc 180
cgtgaacacc gcacccccca ccaaacaaat ttaaatagag ttacggcggc cacagcgac 239

<210> 20
<211> 248
<212> DNA
<213> Mycobacterium avium Serovar 26

<400> 20
cgggaaacct tctccaagat caggtttctc acccttttag agggataagg ccccccgcag 60
accacgggtt cgataggcca gacctggaag ctcagcaatg agtgcaggga actggcacta 120
accggccgaa aacttaccaa cacacaatcg caaccacagt ccatttcggc ggcattttgc 180
cgctggaagc ttgaacaccg caccccccac caaacaaatt taaatagagt tacggcggcc 240
acagcgac 248

<210> 21
<211> 248
<212> DNA
<213> Mycobacterium avium Serovar 28

<400> 21
cgggaaacct tctccaagat caggtttctc acccttttag agggataagg ccccccgcag 60
accacgggtt cgataggcca gacctggaag ctcagcaatg agtgcaggga actggcacta 120
accggccgaa aacttaccaa cacacaatcg caaccacagt ccatttcggc ggcattttgc 180
cgctggaagc ttgaacaccg caccccccac caaacaaatt taaatagagt tacggcggcc 240
acagcgac 248

MYCOBAK.APP

<210> 22
<211> 249
<212> DNA
<213> Mycobacterium avium Serovar 4

<400> 22
cgggaaacct tctccaagat caggtttctc acccttttag agggataagg ccccccgcag 60
accacgggat tgataggcca gacctggaag ctcagtaatg agtgcaggga actggcacta 120
accggccgaa aacttaccaa cacacaactc gcaaccacga tccattttcg cggcaattcg 180
ccgtggaaac tcgggcacca caccccccac cgaaacaaat ttaaatagag ttacggcggc 240
cacagcgac 249

<210> 23
<211> 249
<212> DNA
<213> Mycobacterium avium Serovar 5

<400> 23
cgggaaacct tctccaagat caggtttttc acccttttag agggataagg ccccccgcag 60
accacgggat tgataggcca gacctggaag ctcagtaatg agtgcaggga actggcacta 120
accggccgaa aacttaccaa cacacaactc gcaaccacga tccattttcg cggcaattcg 180
ccgtggaaac tcgggcacca caccccccac cgaaacaaat ttaaatagag ttacggcggc 240
cacagcgac 249

<210> 24
<211> 249
<212> DNA
<213> Mycobacterium avium Serovar 6

<400> 24
cgggaaacct tctccaagat caggtttttc acccttttag agggataagg ccccccgcag 60
accacgggat tgataggcca gacctggaag ctcagtaatg agtgcaggga actggcacta 120
accggccgaa aacttaccaa cacacaactc gcaaccacga tccattttcg cggcaattcg 180
ccgtggaaac tcgggcacca caccccccac cgaaacaaat ttaaatagag ttacggcggc 240
cacagcgac 249

<210> 25
<211> 239
<212> DNA
<213> Mycobacterium avium Serovar 7

<400> 25
cgggaaacct tctccaagat caggtttctc acccttttag agggataagg ccccccgcag 60
accacgggat cgataggcca gacctggaag ctcagtaacg agtgcaggga actggcacta 120
accggccgaa aacttaccaa cacacaatcg caaccacagt ccaattcatg gcttcattgc 180
cgtgaacacc gaacccccca ccaaacaaat ttaaatagag ttacggcggc cacagcgac 239

<210> 26
<211> 249
<212> DNA
<213> Mycobacterium avium Serovar 9

<400> 26
cgggaaacct tctccaagat caggtttctc acccttttag agggataagg ccccccgcag 60
accacgggat tgataggcca gacctggaag ctcagtaatg agtgcaggga actggcacta 120
accggccgaa aacttaccaa cacacaactc gcaaccacga tccattttcg cggcaattcg 180
ccgtggaaac tcgggcacca caccccccac cgaaacaaat ttaaatagag ttacggcggc 240
cacagcgac 249

<210> 27
<211> 263

MYCOBAK.APP
<212> DNA
<213> Mycobacterium bovis Serovar 1

<400> 27
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aacttacaac accctccctt ttggaaaagg gaggcaaaaa caaactcgca 180
accacatccg ttcacggcgc tagccgtgcg tccacacccc ccaccagaac aaatttgcat 240
agagttacgg cggccacagc ggc 263


<210> 28
<211> 263
<212> DNA
<213> Mycobacterium bovis Serovar 2

<400> 28
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aacttacaac accctccctt ttggaaaagg gaggcaaaaa caaactcgca 180
accacatccg ttcacggcgc tagccgtgcg tccacacccc ccaccagaac aaatttgcat 240
agagttacgg cggccacagc ggc 263


<210> 29
<211> 263
<212> DNA
<213> Mycobacterium bovis Serovar 4

<400> 29
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aacttacaac accctccctt ttggaaaagg gaggcaaaaa caaactcgca 180
accacatccg ttcacggcgc tagccgtgcg tccacacccc ccaccagaac aaatttgcat 240
agagttacgg cggccacagc ggc 263


<210> 30
<211> 307
<212> DNA
<213> Mycobacterium fortuitum

<400> 30
cgggaaacct tctccaagat caggcttttc accctttagg agggataagg ccccccgcag 60
accacgggat tgatagacca gacctggaag cccagcaatg ggtgcaggga actggcacta 120
accggccgaa aacttacaac aaccaacaaa caccaaaagt ttgaatggga ttcgtaagaa 180
ccaaattgcc tcgcaaccac accacaaaca cccacacctc gtgtgatcgg agtgcaccgc 240
accccaccac caaaacacaa ggtttacact caacaacgag tgaatagagt tacggcggca 300
cagcgnc 307


<210> 31
<211> 245
<212> DNA
<213> Mycobacterium gordonae

<400> 31
cgggaaacct tctccaagat caggtttctc accctctagg agggataagg cccccagcag 60
aacactggat tgataggcca gacctggaag ctcagtaatg agtgcaggga actggcacta 120
accggccgaa aacttaccaa cacaaccaat cgcaaccact attcgttcgt cgaccgcaag 180
gtcgatcaac accgcacccc gccaccaaac acaaatttaa atagagttac ggcggccaca 240
gcgac 245


<210> 32
<211> 245
<212> DNA
<213> Mycobacterium gordonae ATCC 14470

MYCOBAK.APP

<400> 32
cgggaaacct tctccaagat caggtttctc accctctagg agggataagg cccccagcag 60
aacactggat tgataggcca gacctggaag ctcagtaatg agtgcaggga actggcacta 120
accggccgaa aacttaccaa cacaaccaat cgcaaccact attcgttcgt cgaccgcaag 180
gtcgatcaac accgcacccc gccaccaaac acaaatttaa atagagttac ggcggccaca 240
gcgac 245

<210> 33
<211> 248
<212> DNA
<213> Mycobacterium intracellulare ATCC 13950

<400> 33
cgggaaacct tctccaagat caggtttctc acccttttag agggataagg ccccccgcag 60
accacgggtt cgataggcca gacctggaag ctcagcaatg agtgcaggga actggcacta 120
accggccgaa aacttaccaa cacacaatcg caaccacagt ccatttcggc ggcattttgc 180
cgctggaagc ttgaacaccg caccccccac caaacaaatt taaatagagt tacggcggcc 240
acagcgac 248

<210> 34
<211> 248
<212> DNA
<213> Mycobacterium intracellulare ATCC 35761

<400> 34
cgggaaacct tctccaagat caggtttctc acccttttag agggataagg ccccccgcag 60
accacgggtt cgataggcca gacctggaag ctcagcaatg agtgcaggga actggcacta 120
accggccgaa aacttaccaa cacacaatcg caaccacagt ccatttcggc ggcattttgc 180
cgctggaagc ttgaacaccg caccccccac caaacaaatt taaatagagt tacggcggcc 240
acagcgac 248

<210> 35
<211> 248
<212> DNA
<213> Mycobacterium intracellulare ATCC 35762

<400> 35
cgggaaacct tctccaagat caggtttctc acccttttag agggataagg ccccccgcag 60
accacgggtt cgataggcca gacctggaag ctcagcaatg agtgcaggga actggcacta 120
accggccgaa aacttaccaa cacacaatcg caaccacagt ccatttcggc ggcattttgc 180
cgctggaagc ttgaacaccg caccccccac caaacaaatt taaatagagt tacggcggcc 240
acagcgac 248

<210> 36
<211> 248
<212> DNA
<213> Mycobacterium intracellulare ATCC 35764

<400> 36
cgggaaacct tctccaagat caggtttctc acccttttag agggataagg ccccccgcag 60
accacgggtt cgataggcca gacctggaag ctcagcaatg agtgcaggga actggcacta 120
accggccgaa aacttaccaa cacacaatcg caaccacagt ccatttcggc ggcattttgc 180
cgctggaagc ttgaacaccg caccccccac caaacaaatt taaatagagt tacggcggcc 240
acagcgac 248

<210> 37
<211> 249
<212> DNA
<213> Mycobacterium intracellulare ATCC 35765

<400> 37
cgggaaacct tctccaagat caggtttctc acccttttag agggataagg ccccccgcag 60

MYCOBAK.APP
accacgggat tgataggcca gacctggaag ctcagtaatg agtgcaggga actggcacta 120
accggccgaa aacttaccaa cacacaactc gcaaccacga tccattttcg cggcaattcg 180
ccgtggaaay tcgggcacca caccccccac cgadacaaav vtaaatagag ttacggcggc 240
cacagcgac 249

<210> 38
<211> 249
<212> DNA
<213> Mycobacterium intracellulare ATCC 35766

<400> 38
cgggaaacct tctccargat caggtttctc acccttttag agggataagg ccccccgcag 60
accacgggat tgataggcca gacctggaag ctcagtaatg agtgcaggga actggcacta 120
accggccgaa aacttaccaa cacacaactc gcaaccacga tccattttcg cggcaattcg 180
ccgtggaaac tcgggcacca caccccccac cgaaacaaat ttaaatagag ttacggcggc 240
cacagcgac 249

<210> 39
<211> 249
<212> DNA
<213> Mycobacterium intracellulare ATCC 35774

<400> 39
cgggaaacct tctccaagat caggtttctc acccttttar agggataagg cccccsgcag 60
accacgggat tgataggcca gacctggaag ctcagtaatg agtgcaggga actggcacta 120
accggccgaa aacttaccaa cacacaactc gcaaccacga tccattttcg cggcaattcg 180
ccgtggaaac tcgggcacca caccccccac cgaaacaaay ytaaatagag ttacggcggc 240
cacagcgac 249

<210> 40
<211> 240
<212> DNA
<213> Mycobacterium intracellulare ATCC 35847

<400> 40
cgggaaacct tctccaagat caggtttctc acccttttag agggataagg ccccccgcag 60
accacgggtt cgataggcca gacctggaag ctcagtaatg agtgcaggga actggcacta 120
accggccgaa aacttaccaa cacacaatcg caaccacagt ccatttcacg gcagcaatgc 180
cgcgaaacac cacacccccc accaaacaaa yytaaataga gttacggcgg ccacagcgac 240

<210> 41
<211> 248
<212> DNA
<213> Mycobacterium intracellulare

<400> 41
cgggaaacct tctccaagat caggtttctc acccttttag agggataagg ccccccgcag 60
accacgggtt cgataggcca gacctggaag ctcagcaatg agtgcaggga actggcacta 120
accggccgaa aacttaccaa cacacaatcg caaccacagt ccatttcggc ggcattttgc 180
cgctggaagc ttgaacaccg caccccccac caaacaaatt taaatagagt tacggcggcc 240
acagcgac 248

<210> 42
<211> 240
<212> DNA
<213> Mycobacterium kansasii ATCC 12478

<400> 42
cgggaaacct tctccaagat caggcttctc acccacttgg tgggataagg ccccccgcag 60
aacacgggtt cgataggcca gacctggaag ctcagtaatg agtgaaggga actggcacta 120
accggccgaa aacttaccaa cacaaataat cgcaaccact ttccacgctc acggtgtcag 180
ccgtgagtcc acacccccca ccaatacaaa tttacataga gttacggcgg ccacagcggc 240

MYCOBAK.APP

<210> 43
<211> 240
<212> DNA
<213> Mycobacterium kansasii

<400> 43
cgggaaacct tctccaagat caggcttctc acccacttgg tgggataagg ccccccgcag 60
aacacgggtt cgataggcca gacctggaag ctcagtaatg agtgaaggga actggcacta 120
accggccgaa aacttaccaa cacaaataat cgcaaccact ttccacgctc acggtgtcag 180
ccgtgagtcc acacccccca ccaatacaaa tttacataga gttacggcgg ccacagcggc 240

<210> 44
<211> 263
<212> DNA
<213> Mycobacterium tuberculosis Patientenisolat

<400> 44
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aacttacaac accctccctt ttggaaaagg gaggcaaaaa caaactcgca 180
accacatccg ttcacggcgc ttnccgtgcg tccacacccc ccaccagaac aaatttgcat 240
agagttacgg cggccacagc ggc 263

<210> 45
<211> 263
<212> DNA
<213> Mycobacterium tuberculosis Patientenisolat

<400> 45
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aacttacaac accctccctt ttggaaaagg gaggcaaaaa caaactcgca 180
accacatccg ttcacggcgc tagccgtgcg tccacacccc ccaccagaac aaatttgcat 240
agagttacgg cggccacagc ggc 263

<210> 46
<211> 263
<212> DNA
<213> Mycobacterium tuberculosis Patientenisolat

<400> 46
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aacttacaac accctccctt ttggaaaagg gaggcaaaaa caaactcgca 180
accacatccg ttcacggcgc tagccgtgcg tccacacccc ccaccagaac aaatttgcat 240
agagttacgg cggccacagc ggc 263

<210> 47
<211> 263
<212> DNA
<213> Mycobacterium tuberculosis Patientenisolat

<400> 47
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aacttacaac accctccctt ttggaaaagg gaggcaaaaa caaactcgca 180
accacatccg ttcacggcgc tagccgtgcg tccacacccc ccaccagaac aaatttgcat 240
agagttacgg cggccacagc ggc 263

<210> 48
<211> 263
<212> DNA

MYCOBAK.APP
<213> Mycobacterium tuberculosis Patientenisolat

<400> 48
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aacttacaac accctccctt ttggaaaagg gaggcaaaaa caaactcgca 180
accacatccg ttcacggcgc tagccgtgcg tccacacccc ccaccagaac aaatttgcat 240
agagttacgg cggccacagc ggc 263

<210> 49
<211> 263
<212> DNA
<213> Mycobacterium tuberculosis Patientenisolat

<400> 49
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aacttacaac accctccctt ttggaaaagg gaggcaaaaa caaactcgca 180
accacatccg ttcacggcgc tagccgtgcg tccacacccc ccaccagaac aaatttgcat 240
agagttacgg cggccacagc ggc 263

<210> 50
<211> 251
<212> DNA
<213> Mycobacterium phlei

<400> 50
cgggaaacct tctccaagat caggcttttc accctttagg agggataagg ccccccgcag 60
accacgggat cgatagacca gacctgcacg cacagcaatg tgttcaggga actggcatta 120
accggccgaa aacttacaca caacgttgca accacatcca acagtcacca tgattgcacc 180
ccaccaccaa aacattcccc cccccaacaa caactggtgc acttaaatag agttacggcg 240
gccacagcgn c 251

<210> 51
<211> 305
<212> DNA
<213> Mycobacterium smegmatis

<400> 51
cgggaaacct tctccaagat cagcttttca cccatgggga gggataaggc cccccgcaga 60
acactgaatt ataggtcaga cctggaagct agtaatacgg ttgcagggag aactggcact 120
aaccggccga aaacttacaa caccccataa tcgttgtaga agaaacattg acgyaccgcg 180
actcgggcaa ccacatdcca cggatgatac aaaccgatac agccccacca ccaaaacaaa 240
gcccacaagt ttgctctcat gtgggtcacc aaataagaga atagagttac ggcggtcata 300
gcgga 305

<210> 52
<211> 245
<212> DNA
<213> Mycobacterium szulgai ATCC 35799

<400> 52
cgggaaacct tctccaagat caggcttctc acccttttag agggatatag gcccctccgc 60
agaacacggg atcgataggc cagacctgga agctcagcaa tgagtgaagg gaactggcac 120
taaccggccg aaaacttacc aacacaacaa atcgcaacca caatccattt cacggcttat 180
agccgtaaaa caccgcaccc cgccaccagt acaaayytaa atagagttac ggcggccaca 240
gcgac 245

<210> 53
<211> 246
<212> DNA
<213> Mycobacterium szulgai

MYCOBAK.APP
<400> 53
cgggaaacct tctccaagat caggcttctc acccttttag agggatatag gcccctccgc 60
agaacacggg atcgataggc cagacctgga agctcagcaa tgagtgaagg gaactggcac 120
taaccggccg aaaacttacc aacacaacaa atcgcaacca caatccattt cacggcttga 180
tagccgtaaa acaccgcacc ccgccaccag tacaaattta aatagagtta cggcggccac 240
agcgac 246

<210> 54
<211> 263
<212> DNA
<213> Mycobacterium terrae

<400> 54
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aayttacaac mccctccctt ttggaaaagg gaggcaaaaa caamctcgha 180
accacatccg ttcacggcgc tagccgtgcg tccacacccc ccaccagaac aaatttgcat 240
agagttacgg cggccacagc gnc 263

<210> 55
<211> 263
<212> DNA
<213> Mycobacterium xenopi

<400> 55
cgggaaacct tctccaagat caggtttctc acccacttgg tgggataagg ccccccgcag 60
aacacgggtt caataggtca gacctggaag ctcagtaatg ggtgtaggga actggtgcta 120
accggccgaa aacttacaac accctccctt ttggaaaagg gaggcaaaaa caaactcgca 180
accacatccg ttcacggcgc tagccgtgcg tccacacccc ccaccagaac aaatttgcat 240
agagttacgg cggccacagc ggc 263

<210> 56
<211> 19
<212> DNA
<213> Künstliche Sequenz

<220>
<223> Beschreibung der künstlichen Sequenz:
gattungsspezifischer Primer MCON1

<400> 56
cgggaaacct tctccaaga 19

<210> 57
<211> 20
<212> DNA
<213> Künstliche Sequenz

<220>
<223> Beschreibung der künstlichen Sequenz:
gattungsspezifischer Primer MCON2c

<400> 57
tgtggccgcc gtaactctat 20

<210> 58
<211> 18
<212> DNA
<213> Künstliche Sequenz

<220>
<223> Beschreibung der künstlichen Sequenz: Primer MTC1

MYCOBAK.APP
<400> 58
ttggaaaagg gaggcaaa 18

<210> 59
<211> 18
<212> DNA
<213> Künstliche Sequenz

<220>
<223> Beschreibung der künstlichen Sequenz: Primer MTC2

<400> 59
tttgcctccc ttttccaa 18

<210> 60
<211> 12
<212> DNA
<213> Künstliche Sequenz

<220>
<223> Beschreibung der künstlichen Sequenz: Primer
MAI-1c

<400> 60
cggcaaaatg cc 12

<210> 61
<211> 12
<212> DNA
<213> Künstliche Sequenz

<220>
<223> Beschreibung der künstlichen Sequenz: Primer
MAI-2c

<400> 61
cggcgaattg cc 12

<210> 62
<211> 18
<212> DNA
<213> Künstliche Sequenz

<220>
<223> Beschreibung der künstlichen Sequenz: Primer
INT35772

<400> 62
atgaagccat gaattgga 18

<210> 63
<211> 30
<212> DNA
<213> Künstliche Sequenz

<220>
<223> Beschreibung der künstlichen Sequenz: Primer
MCONpro1

<400> 63
acacccccca ccagaacaaa tttaaataga 30

MYCOBAK.APP
<210> 64
<211> 31
<212> DNA
<213> Künstliche Sequenz

<220>
<223> Beschreibung der künstlichen Sequenz: Primer
      MCONpro2

<400> 64
agggaactgg tgctaaccgg ccgaaaactt a                                    31


<210> 65
<211> 27
<212> DNA
<213> Künstliche Sequenz

<220>
<223> Beschreibung der künstlichen Sequenz: Primer
      MTCpro1

<400> 65
cccttttgga aaagggaggc aaaaaca                                         27


<210> 66
<211> 29
<212> DNA
<213> Künstliche Sequenz

<220>
<223> Beschreibung der künstlichen Sequenz: Primer
      MTCpro2

<400> 66
aactcgcaac cacatccgtt cacggcgct                                       29


<210> 67
<211> 35
<212> DNA
<213> Künstliche Sequenz

<220>
<223> Beschreibung der künstlichen Sequenz: Primer
      MTCpro3

<400> 67
tgctaaccgg ccgaaaactt acaacaccct ccctt                               35


<210> 68
<211> 24
<212> DNA
<213> Künstliche Sequenz

<220>
<223> Beschreibung der künstlichen Sequenz: Primer
      INT35772pro

<400> 68
tccaattcat ggcttcattg ccgt                                            24


<210> 69
<211> 26
<212> DNA

MYCOBAK.APP
<213> Künstliche Sequenz

<220>
<223> Beschreibung der künstlichen Sequenz: Primer
      MAIpro1

<400> 69
cacagtccat ttcggcggca ttttgc                                          26


<210> 70
<211> 28
<212> DNA
<213> Künstliche Sequenz

<220>
<223> Beschreibung der künstlichen Sequenz: Primer
      MAIpro2

<400> 70
cacgatccat tttcgcggca attcgccg                                        28


<210> 71
<211> 28
<212> DNA
<213> Künstliche Sequenz

<220>
<223> Beschreibung der künstlichen Sequenz: Primer
      INT35772pro1

<400> 71
ccggccgaaa acttaccaac acacaatc                                        28


<210> 72
<211> 250
<212> DNA
<213> Künstliche Sequenz

<220>
<223> Beschreibung der künstlichen Sequenz:
      Mehrheitssequenz (Konsensussequenz) der
      23S-5S-Spacerregion des MAI-Komplexes

<400> 72
cgggaaacct tctccaagat caggtttctc acccttttag agggataagg ccccccgcag 60
accacgggat tgataggcca gacctggaag ctcagtaatg agtgcaggga actggcacta 120
accggccgaa aacttaccaa cacacaactc gcaaccacgg tccatttccg cggcaattcg 180
ccgctggaaa ctcgggcacc acacccccca ccgaaacaaa tttaaataga gttacggcgg 240
ccacagcgac                                                            250

E) PCR-Primer und Detektionssonden
MCON1 (19mer): 5´ CGGGAAACCTTCTCCAAGA 3´ SEQ.ID.NO.56
MCON2c (20mer): 5´ TGTGGCCGCCGTAACTCTAT 3´ SEQ.ID.NO.57
MTC1 (18mer): 5´ TTG GAA AAG GGA GGC AAA 3´ SEQ.ID.NO.58
MTC2 (18mer): 5´ TTT GCC TCC CTT TTC CAA 3´ SEQ.ID.NO.59
MAI-1c (12mer): 5´ CGG CAA AAT GCC 3´ SEQ.ID.NO.60
MAI-2c (12mer): 5´ CGG CGA ATT GCC 3´ SEQ.ID.NO.61
INT35772 (18mer): 5´ ATG AAG CCA TGA ATT GGA 3´ SEQ.ID.NO.62
MCONpro1 (30mer): 5´ACACCCCCACCAGAACAAATTTAAATAGA3´ SEQ.ID.NO.63
MCONpro2 (31mer):5´ AGGGAACTGGTGCTAACCGGCCGAAAACTTA 3´
SEQ.ID.NO.64
MTCpro1 (27mer): 5´ CCC TTT TGG AAA AGG GAG GCA AAA ACA 3´ SEQ.ID.NO.65
MTCpro2 (29mer): 5´ AAC TCG CAA CCA CAT CCG TTC ACG GCG CT 3´
SEQ.ID.NO.66

MTCpro3 (35mer):5´TGCTAACCGGCCGAAAACTTACAACACCCTCCCTT3´
SEQ.ID.NO.67
INT35772pro (24mer):5´ TCC AAT TCA TGG CTT CAT TGC CGT 3´ SEQ.ID.NO.68
MAIpro1 (26mer): 5´ CAC AGT CCA TTT CGG CGG CAT TTT GC 3´ SEQ.ID.NO.69
MAIpro2 (28mer): 5´ CAC GAT CCA TTT TCG CGG CAA TTC GCC G 3´ SEQ.ID.NO.70
INT35772pro1 (28mer): 5´ CCGGCCGAAAACTTACCAACACACAATC 3´ SEQ.ID.NO.71

SEQ.ID.NO.1 (MEHRHEITSSEQUENZ, POSITION 1-318)
5′ CGG GAA ACC TTC TCC AAG ATC AGG TTT CTC ACC CTT TTA GAG GGA
TAA GGC CCC CC- GCA GAC CAC GGG TTC GAT AGG CCA GAC CTG GAA GCT
CAG TAA T-- GAG TGC AGG --G AAC TGG CAC TAA CCG GCC GAA AAC TTA
CCA -AC ACA -CA A-T CGC AAC --- --- --- --- --- --C ACA ATC CAT --- --T TCG --A
CGG CAT TTC G-- -CC GTC G-G AAG C-- --T --- CGT -GC A-- CCA CAC CCC -CC -
AC CAA -A- AC- AAA TTT A-- --- --- --- --- --- -AA TAG AGT TAC GGC GGC CAC
AGC GAC 3′

SEQ.ID.NO.2 (Mehrheitssequenz der partiellen 3´-Region des 23S-Genabschnittes,
Position 1 – 138 der SEQ.ID.NO.1)
5´ CGG GAA ACC TTC TCC AAG ATC AGG TTT CTC ACC CTT TTA GAG GGA
TAA GGC CCC CC- GCA GAC CAC GGG TTC GAT AGG CCA GAC CTG GAA GCT
CAG TAA T-- GAG TGC AGG --G AAC TGG CAC TAA CCG GCC GAA AAC 3´

SEQ.ID.NO.3 (Mehrheitssequenz der partiellen 5´-Region des 5S-Genabschnittes,
Position 294-318 der SEQ.ID.NO.1)
5´A TAG AGT TAC GGC GGC CAC AGC GAC 3´

SEQ.ID.NO.4 (Mehrheitssequenz der 23S-5S-Spacerregion, Position 139-294 der
SEQ.ID.NO.1)
TTA CCA -AC ACA -CA A-T CGC AAC --- --- --- --- --- --C ACA ATC CAT --- --T
TCG --A CGG CAT TTC G-- -CC GTC G-G AAG C-- --T --- CGT -GC A-- CCA CAC
CCC -CC -AC CAA -A- AC- AAA TTT A-- --- --- --- --- --- -A 3´

SEQ.ID.NO.5 (M.tb ATCC 27294)
MTB27294.seq (263bp)
5′CGGGAAACCT  TCTCCAAGAT CAGGTTTCTC ACCCACTTGG TGGGATAAGG
CCCCCCGCAG AACACGGGTT CAATAGGTCA GACCTGGAAG CTCAGTAATG
GGTGTAGGGA ACTGGTGCTA ACCGGCCGAA AACTTACAAC ACCCTCCCTT
TTGGAAAAGG GAGGCAAAAA CAAACTCGCA ACCACATCCG TTCACGGCGC
TAGCCGTGCG TCCACACCCC CCACCAGAAC ACATTTGCAT AGAGTTACGG
CGGCCACAGC GGC

SEQ.ID.NO.6 (M. intracellulare ATCC 35772)
INT35772.seq (239bp)
5´CGGGAAACCT TCTCCAAGAT CAGGTTTCTC ACCCTTTTAG AGGGATAAGG
CCCCCCGCAG ACCACGGGAT CGATAGGCCA GACCTGGAAG CTCAGTAATG
AGTGCAGGGA ACTGGCACTA ACCGGCCGAA AACTTACCAA CACACAATCG
CAACCACAGT CCAATTCATG GCTTCATTGC CGTGAACACC GCACCCCCCA
CCAAACAAAT TTAAATAGAG TTACGGCGGC CACAGCGAC 3´

SEQ.ID.NO.7 (M. bovis ATCC 27289)
BOV27289.seq (263bp)
5´CGGGAAACCTTCTCCAAGATCAGGTTTCTCACCCACTTGGTGGGATAAGGCC
CCCCGCAGAACACGGGTTCAATAGGTCAGACCTGGAAGCTCAGTAATGGGTGT
AGGGAACTGGTGCTAACCGGCCGAAAACTTACAACACCCTCCCTTTTGGAAAA
GGGAGGCAAAAACAAACTCGCAACCACATCCGTTCACGGCGCTAGCCGTGCGT
CCACACCCCCCACCAGAACAAATTTGCATAGAGTTACGGCGGCCACAGCGGC
3´

SEQ.ID.NO.8 (M. avium ATCC 35771)
AVI35771.seq (249bp)
5´CGGGAAACCT TCTCMAARAT AAGGTTTCTM ACCCTTTTAR AGGGATAAGG
CCCCCCGMAG ACCACGGGAT TGATAGGCCA GACCTGGAAG CTCAGTAATG
AGTGCAGGGA ACTGGCACTA ACCGGCCGAA AACTTACCAA CACACAACTC

GCAACCACGA TCCATTTTCG CGGCAATTCG CCGTGGAAAC TCGGGCACCA
CACCCCCCAC CGADACAAAB BTAAATAGAG TTACGGCGGCCACAGCGAC 3′

SEQ.ID.NO.9 (M. intracellulare ATCC 35763)
INT35763.seq (248bp)
5´CGGGAAACCT TCTCCAAGAT CAGGTTTCTC ACCCTTTTAG AGGGATAAGG
CCCCCCGCAG ACCACGGGTT CGATAGGCCA GACCTGGAAG CTCAGCAATG
AGTGCAGGGA ACTGGCACTA ACCGGCCGAA AACTTACCAA CACACAATCG
CAACCACAGT CCATTTCGGC GGCATTTTGC CGCTGGAAGC TTGAACACCG
CACCCCCCAC CAAACAAATT TAAATAGAGT TACGGCGGCC ACAGCGAC 3´

SEQ.ID.NO.10 (M. africanum, ATCC 25420)
AFR25420.seq (263bp)
5´CGGGAAACCTTCTCCAAGATCAGGTTTCTCACCCACTTGGTGGGATAAGGCC
CCGCAGAACACGGGTTCAATAGGTCAGACCTGGAAGCTCAGTAATGGGTGTAG
GGAACTGGTGCTAACCGGCCGAAAACTTACAACACCCTCCCTTTTGGAAAAGG
GAGGCAAAAACAAACTCGCAACCACATCCGTTCACGGCGCTAGCCGTGCGTCC
ACACCCCCCACCAGAACAAATTTGCATAGAGTTACGGCGGCCACAGCGGC

SEQ.ID.NO.11 (M. africanum)
AFRICA.seq (263bp)
5′CGGGAAACCTTCTCCAAGATCAGGTTTCTCACCCACTTGGTGGGATAAGGCC
CCCCGCAGAACACGGGTTCAATAGGTCAGACCTGGAAGCTCAGTAATGGGTGT
AGGGAACTGGTGCTAACCGGCCGAAAACTTACAACACCCTCCCTTTTGGAAAA
GGGAGGCAAAAACAAACTCGCAACCACATCCGTTCACGGCGCTAGCCGTGCGT
CCACACCCCCCACCAGAACAAATTTGCATAGAGTTACGGCGGCCACAGCGGC
3′

SEQ.ID.NO.12 (M. avium, ATCC 15769)
AVI15769.seq (249bp)
5´CGGGAAACCTTCTCCAAGATCAGGTTTCTCACCCTTTTAGAGGGATAAGGCC
CCCCGCAGACCACGGGATTGATAGGCCAGACCTGGAAGCTCAGTAATGAGTGC

AGGGAACTGGCACTAACCGGCCGAAAACTTACCAACACACAACTCGCAACCA
CGATCCATTTTCGCGGCAATTCGCCGTGGAAACTCGGGCACCACACCCCCCAC
CGANACAAATTTAAATAGAGTTACGGCGGCCACAGCGAC 3′

SEQ.ID.NO.13 (M. avium, ATCC 19698)
AVI19698.seq (249bp)
5´CGGGNNACCTTCTCCARGATCAGGTTTCTCACCCTTTTAGAGGGATAAGGCC
CCCCGCAGACCACGGGATTGATAGGCCAGACCTGGAAGCTCAGTAATGAGTGC
AGGGAACTGGCACTAACCGGCCGAAAACTTACCAACACACAACTCGCAACCA
CGATCCATTTTCGCGGCAATTCGCCGTGGAAACTCGGGCACCACACCCCCCAC
CGAAACAAATTTAAATAGAGTTACGGCGGCCACAGCGAC 3´

SEQ.ID.NO.14 (M. avium, ATCC 35712)
AVI35712.seq (249bp)
5´CGGGAAACCTTCTCCAAGATCAGGTTTCTCACCCTTTTAGAGGGATAAGGCC
CCCCGCAGACCACGGGATTGATAGGCCAGACCTGGAAGCTCAGTAATGAGTGC
AGGGAACTGGCACTAACCGGCCGAAAACTTACCAACACACAACTCGCAACCA
CGATCCATTTTCGCGGCAATTCGCCGTGGAAACTCGGGCACCACACCCCCCAC
CGAAACAAATTTAAATAGAGTTACGGCGGCCACAGCGAC 3´

SEQ.ID.NO.15 (M. avium, ATCC 35718)
AVI35718.seq (249bp)
5´CGGGAAACCTTCTCCMAGATCAGGTTTCTCACCCTTTTAGAGGGATAAGGCC
CCCCGCAGACCACGGGATTGATAGGCCAGACCTGGAAGCTCAGTAATGAGTGC
AGGGAACTGGCACTAACCGGCCGAAAACTTACCAACACACAACTCGCAACCA
CGATCCATTTTCGCGGCAATTCGCCGTGGAAACTCGGGCACCACACCCCCCAC
CGAAACAAATTTAAATAGAGTTACGGCGGCCACAGCGAC 3´

SEQ.ID.NO.16 (M. avium, ATCC 35768)
AVI35768.seq (249bp)
5´CGGGAAACCTTCTCCAAGATCAGGTTTCTCACCCTTTTAGAGGGATAAGGCC
CCCCGCAGACCACGGGATTGATAGGCCAGACCTGGAAGCTCAGTAATGAGTGC

AGGGAACTGGCACTAACCGGCCGAAAACTTACCAACACACAACTCGCAACCA
CGATCCATTTTCGCGGCAATTCGCCGTVVMAAMWMVGGCACCAMACCCCCCA
CCGAAACAAATTTAAATAGAGTTACGGCGGCCACAGCGAC 3′

SEQ.ID.NO.17 (M. avium, Serovar 1)
AVIUM1.seq (249bp)
5´CGGGAAACCTTCTCCAAGATCAGGTTTCTCACCCTTTTAGAGGGATAAGGCC
CCCCGCAGACCACGGACTTGATAGGCCAGACCTGGAAGCTCAGTAATGAGTGC
AGGGAACTGGCACTAACCGGCCGAAAACTTACCAACACACAACTCGCAACCA
AGATCCATAACCMCVGCAATTCGCCGTGGAAACCCGGSCACCACACCCCCCAC
CGAAACAAATTTAAATAGAGTTACGGCGGCCACAGCGAC 3´

SEQ.ID.NO.18 (M. avium, Serovar 16)
AVIUM16.seq (248bp)
5´CGGGAAACCTTCTCCAAGATCAGGTTTCTCACCCTTTTAGAGGGATAAGGCC
CCCCGCAGACCACGGGTTCGATAGGCCAGACCTGGAAGCTCAGCARTGAGTGC
AGGGAACTGGCACTAACCGGCCGAAAACTTACCAACACACAATCGCAACCAC
AGTCCATTTCGGCGGCATTTTGCCGCTGGAAGCTTGAACACCGCACCCCCCAC
CAAACAAATTTAAATAGAGTTACGGCGGCCACAGCGAC

SEQ.ID.NO.19 (M. avium, Serovar 19)
AVIUM19.seq (239bp)
5´CGGGAAACCTTCTCCAAGATCAGGTTTCTCACCCTTTTAGAGGGATAAGGCC
CCCCGCAGACCACGGGATCGATAGGCCAGACCTGGAAGCTCAGCAATGAGTG
CAGGGAACTGGCACTAACCGGCCGAAAACTTACCAACACACAATCGCAACCA
CAGTCCAATTCATGGCTTCATTGCCGTGAACACCGCACCCCCCACCAAACAAA
TTTAAATAGAGTTACGGCGGCCACAGCGAC 3´

SEQ.ID.NO.20 (M. avium, Serovar 26)
AVIUM26.seq (248bp)
5´CGGGAAACCTTCTCCAAGATCAGGTTTCTCACCCTTTTAGAGGGATAAGGCC
CCCCGCAGACCACGGGTTCGATAGGCCAGACCTGGAAGCTCAGCAATGAGTGC

AGGGAACTGGCACTAACCGGCCGAAAACTTACCAACACACAATCGCAACCAC
AGTCCATTTCGGCGGCATTTTGCCGCTGGAAGCTTGAACACCGCACCCCCCAC
CAAACAAATTTAAATAGAGTTACGGCGGCCACAGCGAC 3´

SEQ.ID.NO.21 (M. avium, Serovar 28)
AVIUM28.seq (248bp)
5´CGGGAAACCTTCTCCAAGATCAGGTTTCTCACCCTTTTAGAGGGATAAGGCC
CCCCGCAGACCACGGGTTCGATAGGCCAGACCTGGAAGCTCAGCAATGAGTGC
AGGGAACTGGCACTAACCGGCCGAAAACTTACCAACACACAATCGCAACCAC
AGTCCATTTCGGCGGCATTTTGCCGCTGGAAGCTTGAACACCGCACCCCCCAC
CAAACAAATTTAAATAGAGTTACGGCGGCCACAGCGAC 3´

SEQ.ID.NO.22 (M. avium, Serovar 4)
AVIUM4.seq (249bp)
5´CGGGAAACCTTCTCCAAGATCAGGTTTCTCACCCTTTTAGAGGGATAAGGCC
CCCCGCAGACCACGGGATTGATAGGCCAGACCTGGAAGCTCAGTAATGAGTGC
AGGGAACTGGCACTAACCGGCCGAAAACTTACCAACACACAACTCGCAACCA
CGATCCATTTTCGCGGCAATTCGCCGTGGAAACTCGGGCACCACACCCCCCAC
CGAAACAAATTTAAATAGAGTTACGGCGGCCACAGCGAC 3´

SEQ.ID.NO.23 (M. avium, Serovar 5)
AVIUM5.seq (249bp)
5´CGGGAAACCTTCTCCAAGATCAGGTTTTTCACCCTTTTAGAGGGATAAGGCC
CCCCGCAGACCACGGGATTGATAGGCCAGACCTGGAAGCTCAGTAATGAGTGC
AGGGAACTGGCACTAACCGGCCGAAAACTTACCAACACACAACTCGCAACCA
CGATCCATTTTCGCGGCAATTCGCCGTGGAAACTCGGGCACCACACCCCCCAC
CGAAACAAATTTAAATAGAGTTACGGCGGCCACAGCGAC 3´

SEQ.ID.NO.24 (M. avium, Serovar 6)
AVIUM6.seq (249bp)
5´CGGGAAACCTTCTCCAAGATCAGGTTTTTCACCCTTTTAGAGGGATAAGGCC
CCCCGCAGACCACGGGATTGATAGGCCAGACCTGGAAGCTCAGTAATGAGTGC

AGGGAACTGGCACTAACCGGCCGAAAACTTACCAACACACAACTCGCAACCA
CGATCCATTTTCGCGGCAATTCGCCGTGGAAACTCGGCACCACACCCCCCAC
CGAAACAAATTTAAATAGAGTTACGGCGGCCACAGCGAC3´

SEQ.ID.NO.25 (M. avium, Serovar 7)
AVIUM7.seq (239bp)
5´CGGGAAACCTTCTCCAAGATCAGGTTTCTCACCCTTTTAGAGGGATAAGGCC
CCCCGCAGACCACGGGATCGATAGGCCAGACCTGGAAGCTCAGTAACGAGTG
CAGGGAACTGGCACTAACCGGCCGAAAACTTACCAACACACAATCGCAACCA
CAGTCCAATTCATGGCTTCATTGCCGTGAACACCGAACCCCCCACCAAACAAA
TTTAAATAGAGTTACGGCGGCCACAGCGAC 3´

SEQ.ID.NO.26 (M. avium, Serovar 9)
AVIUM9.seq (249bp)
5´CGGGAAACCTTCTCCAAGATCAGGTTTCTCACCCTTTTAGAGGGATAAGGCC
CCCCGCAGACCACGGGATTGATAGGCCAGACCTGGAAGCTCAGTAATGAGTGC
AGGGAACTGGCACTAACCGGCCGAAAACTTACCAACACACAACTCGCAACCA
CGATCCATTTTCGCGGCAATTCGCCGTGGAAACTCGGGCACCACACCCCCCAC
CGAAACAAATTTAAATAGAGTTACGGCGGCCACAGCGAC 3´

SEQ.ID.NO.27 (M. bovis, Serovar 1)
BOVIS1.seq (263bp)
5´CGGGAAACCTTCTCCAAGATCAGGTTTCTCACCCACTTGGTGGGATAAGGCC
CCCCGCAGAACACGGGTTCAATAGGTCAGACCTGGAAGCTCAGTAATGGGTGT
AGGGAACTGGTGCTAACCGGCCGAAAACTTACAACACCCTCCCTTTTGGAAAA
GGGAGGCAAAAACAAACTCGCAACCACATCCGTTCACGGCGCTAGCCGTGCGT
CCACACCCCCCACCAGAACAAATTTGCATAGAGTTACGGCGGCCACAGCGGC
3´

SEQ.ID.NO.28 (M. bovis, Serovar 2)
BOVIS2.seq (263bp)
5′CGGGAAACCTTCTCCAAGATCAGGTTTCTCACCCACTTGGTGGGATAAGGCC
CCCCGCAGAACACGGGTTCAATAGGTCAGACCTGGAAGCTCAGTAATGGGTGT
AGGGAACTGGTGCTAACCGGCCGAAAACTTACAACACCCTCCCTTTTGGAAAA
GGGAGGCAAAAACAAACTCGCAACCACATCCGTTCACGGCGCTAGCCGTGCGT
CCACACCCCCCACCAGAACAAATTTGCATAGAGTTACGGCGGCCACAGCGGC
3′

SEQ.ID.NO.29 (M. bovis, Serovar 4)
BOVIS4.seq (263bp)
5′CGGGAAACCTTCTCCAAGATCAGGTTTCTCACCCACTTGGTGGGATAAGGCC
CCCCGCAGAACACGGGTTCAATAGGTCAGACCTGGAAGCTCAGTAATGGGTGT
AGGGAACTGGTGCTAACCGGCCGAAAACTTACAACACCCTCCCTTTGGAAAA
GGGAGGCAAAAACAAACTCGCAACCACATCCGTTCACGGCGCTAGCCGTGCGT
CCACACCCCCCACCAGAACAAATTTGCATAGAGTTACGGCGGCCACAGCGGC
3′

SEQ.ID.NO.30 (M. fortuitum)
FOR7360.seq (307bp)
5′CGGGAAACCTTCTCCAAGATCAGGCTTTTCACCCTTTAGGAGGGATAAGGCC
CCCCGCAGACCACGGGATTGATAGACCAGACCTGGAAGCCCAGCAATGGGTG
CAGGGAACTGGCACTAACCGGCCGAAAACTTACAACAACCAACAAACACCAA
AAGTTTGAATGGGATTCGTAAGAACCAAATTGCCTCGCAACCACACCACAAAC
ACCCACACCTCGTGTGATCGGAGTGCACCGCACCCCACCACCAAAACACAAGG
TTTACACTCAACAACGAGTGAATAGAGTTACGGCGGCACAGCGNC 3′

SEQ.ID.NO.31 (M. gordonae)
GORDON.seq (245bp)
5′CGGGAAACCTTCTCCAAGATCAGGTTTCTCACCCTCTAGGAGGGATAAGGCC
CCCAGCAGAACACTGGATTGATAGGCCAGACCTGGAAGCTCAGTAATGAGTGC
AGGGAACTGGCACTAACCGGCCGAAAACTTACCAACACAACCAATCGCAACC

ACTATTCGTTCGTCGACCGCAAGGTCGATCAACACCGCACCCCGCCACCAAAC
ACAAATTTAAATAGAGTTACGGCGGCCACAGCGAC 3′

SEQ.ID.NO.32 (M. gordonae, ATCC 14470)
GOR14470.seq (245bp)
5′CGGGAAACCTTCTCCAAGATCAGGTTTCTCACCCTCTAGGAGGGATAAGGCC
CCCAGCAGAACACTGGATTGATAGGCCAGACCTGGAAGCTCAGTAATGAGTGC
AGGGAACTGGCACTAACCGGCCGAAAACTTACCAACACAACCAATCGCAACC
ACTATTCGTTCGTCGACCGCAAGGTCGATCAACACCGCACCCCGCCACCAAAC
ACAAATTTAAATAGAGTTACGGCGGCCACAGCGAC 3′

SEQ.ID.NO.33 (M. intrazellulare, ATCC 13950)
INT13950.seq (248bp)
5´CGGGAAACCTTCTCCAAGATCAGGTTTCTCACCCTTTTAGAGGGATAAGGCC
CCCCGCAGACCACGGGTTCGATAGGCCAGACCTGGAAGCTCAGCAATGAGTGC
AGGGAACTGGCACTAACCGGCCGAAAACTTACCAACACACAATCGCAACCAC
AGTCCATTTCGGCGGCATTTTGCCGCTGGAAGCTTGAACACCGCACCCCCCAC
CAAACAAATTTAAATAGAGTTACGGCGGCCACAGCGAC 3´

SEQ.ID.NO.34 (M. intrazellulare, ATCC 35761)
INT35761.seq (248bp)
5´CGGGAAACCTTCTCCAAGATCAGGTTTCTCACCCTTTTAGAGGGATAAGGCC
CCCCGCAGACCACGGGTTCGATAGGCCAGACCTGGAAGCTCAGCAATGAGTGC
AGGGAACTGGCACTAACCGGCCGAAAACTTACCAACACACAATCGCAACCAC
AGTCCATTTCGGCGGCATTTTGCCGCTGGAAGCTTGAACACCGCACCCCCCAC
CAAACAAATTTAAATAGAGTTACGGCGGCCACAGCGAC 3´

SEQ.ID.NO.35 (M. intrazellulare, ATCC 35762)
INT35762.seq (248bp)
5´CGGGAAACCTTCTCCAAGATCAGGTTTCTCACCCTTTTAGAGGGATAAGGCC
CCCCGCAGACCACGGGTTCGATAGGCCAGACCTGGAAGCTCAGCAATGAGTGC
AGGGAACTGGCACTAACCGGCCGAAAACTTACCAACACACAATCGCAACCAC

AGTCCATTTCGGCGGCATTTTGCCGCTGGAAGCTTGAACACCGCACCCCCCAC
CAAACAAATTTAAATAGAGTTACGGCGGCCACAGCGAC 3'

SEQ.ID.NO.36 (M. intrazellulare, ATCC 35764)
INT35764.seq (248bp)
5´CGGGAAACCTTCTCCAAGATCAGGTTTCTCACCCTTTTAGAGGGATAAGGCC
CCCCGCAGACCACGGGTTCGATAGGCCAGACCTGGAAGCTCAGCAATGAGTGC
AGGGAACTGGCACTAACCGGCCGAAAACTTACCAACACACAATCGCAACCAC
AGTCCATTTCGGCGGCATTTTGCCGCTGGAAGCTTGAACACCGCACCCCCCAC
CAAACAAATTTAAATAGAGTTACGGCGGCCACAGCGAC 3´

SEQ.ID.NO.37 (M. intrazellulare, ATCC 35765)
INT35765.seq (249bp)
5´CGGGAAACCTTCTCCAAGATCAGGTTTCTCACCCTTTTAGAGGGATAAGGCC
CCCCGCAGACCACGGGATTGATAGGCCAGACCTGGAAGCTCAGTAATGAGTGC
AGGGAACTGGCACTAACCGGCCGAAAACTTACCAACACACAACTCGCAACCA
CGATCCATTTTCGCGGCAATTCGCCGTGGAAAYTCGGGCACCACACCCCCCAC
CGADACAAAVVTAAATAGAGTTACGGCGGCCACAGCGAC 3´

SEQ.ID.NO.38 (M. intrazellulare, ATCC 35766)
INT35766.seq (249bp)
5´CGGGAAACCTTCTCCARGATCAGGTTTCTCACCCTTTTAGAGGGATAAGGCC
CCCCGCAGACCACGGGATTGATAGGCCAGACCTGGAAGCTCAGTAATGAGTGC
AGGGAACTGGCACTAACCGGCCGAAAACTTACCAACACACAACTCGCAACCA
CGATCCATTTTCGCGGCAATTCGCCGTGGAAACTCGGGCACCACACCCCCCAC
CGAAACAAATTTAAATAGAGTTACGGCGGCCACAGCGAC 3´

SEQ.ID.NO.39 (M. intrazellulare, ATCC 35774)
INT35774.seq (249bp)
5´CGGGAAACCTTCTCCAAGATCAGGTTTCTCACCCTTTTARAGGGATAAGGCC
CCCSGCAGACCACGGGATTGATAGGCCAGACCTGGAAGCTCAGTAATGAGTGC
AGGGAACTGGCACTAACCGGCCGAAAACTTACCAACACACAACTCGCAACCA

CGATCCATTTTCGCGGCAATTCGCCGTGGAAACTCGGGCACCACACCCCCCAC
CGAAACAAAYYTAAATAGAGTTACGGCGGCCACAGCGAC 3′

SEQ.ID.NO.40 (M. intrazellulare, ATCC 35847)
INT35847.seq (240bp)
5´CGGGAAACCTTCTCCAAGATCAGGTTTCTCACCCTTTTAGAGGGATAAGGCC
CCCCGCAGACCACGGGTTCGATAGGCCAGACCTGGAAGCTCAGTAATGAGTGC
AGGGAACTGGCACTAACCGGCCGAAAACTTACCAACACACAATCGCAACCAC
AGTCCATTTCACGGCAGCAATGCCGCGAAACACCACACCCCCCACCAAACAAA
YYTAAATAGAGTTACGGCGGCCACAGCGAC 3´

SEQ.ID.NO.41 (M. intrazellulare)
INTRA.seq (248bp)
5´CGGGAAACCTTCTCCAAGATCAGGTTTCTCACCCTTTTAGAGGGATAAGGCC
CCCCGCAGACCACGGGTTCGATAGGCCAGACCTGGAAGCTCAGCAATGAGTGC
AGGGAACTGGCACTAACCGGCCGAAAACTTACCAACACACAATCGCAACCAC
AGTCCATTTCGGCGGCATTTTGCCGCTGGAAGCTTGAACACCGCACCCCCCAC
CAAACAAATTTAAATAGAGTTACGGCGGCCACAGCGAC 3´

SEQ.ID.NO.42 (M. kansasii, ATCC 12478)
KAN12478.seq (240bp)
5´CGGGAAACCTTCTCCAAGATCAGGCTTCTCACCCACTTGGTGGGATAAGGCC
CCCCGCAGAACACGGGTTCGATAGGCCAGACCTGGAAGCTCAGTAATGAGTGA
AGGGAACTGGCACTAACCGGCCGAAAACTTACCAACACAAATAATCGCAACC
ACTTTCCACGCTCACGGTGTCAGCCGTGAGTCCACACCCCCCACCAATACAAA
TTTACATAGAGTTACGGCGGCCACAGCGGC 3´

SEQ.ID.NO.43 (M. kansasii)
KANB180.seq (240bp)
5′CGGGAAACCTTCTCCAAGATCAGGCTTCTCACCCACTTGGTGGGATAAGGCC
CCCCGCAGAACACGGGTTCGATAGGCCAGACCTGGAAGCTCAGTAATGAGTGA
AGGGAACTGGCACTAACCGGCCGAAAACTTACCAACACAAATAATCGCAACC

ACTTTCCACGCTCACGGTGTCAGCCGTGAGTCCACACCCCCACCAATACAAA
TTTACATAGAGTTACGGCGGCCACAGCGGC 3′

SEQ.ID.NO.44 (M. tuberculosis, Patientenisolat)
MTB-P02.seq (263bp)
5´CGGGAAACCTTCTCCAAGATCAGGTTTCTCACCCACTTGGTGGGATAAGGCC
CCCCGCAGAACACGGGTTCAATAGGTCAGACCTGGAAGCTCAGTAATGGGTGT
AGGGAACTGGTGCTAACCGGCCGAAAACTTACAACACCCTCCCTTTTGGAAAA
GGGAGGCAAAAACAAACTCGCAACCACATCCGTTCACGGCGCTTNCCGTGCGT
CCACACCCCCCACCAGAACAAATTTGCATAGAGTTACGGCGGCCACAGCGGC
3´

SEQ.ID.NO.45 (M. tuberculosis, Patientenisolat)
MTB-P03.seq (263bp)
5´CGGGAAACCTTCTCCAAGATCAGGTTTCTCACCCACTTGGTGGGATAAGGCC
CCCCGCAGAACACGGGTTCAATAGGTCAGACCTGGAAGCTCAGTAATGGGTGT
AGGGAACTGGTGCTAACCGGCCGAAAACTTACAACACCCTCCCTTTTGGAAAA
GGGAGGCAAAAACAAACTCGCAACCACATCCGTTCACGGCGCTAGCCGTGCGT
CCACACCCCCCACCAGAACAAATTTGCATAGAGTTACGGCGGCCACAGCGGC
3´

SEQ.ID.NO.46 (M. tuberculosis, Patientenisolat)
MTB-P05.seq (263bp)
5´CGGGAAACCTTCTCCAAGATCAGGTTTCTCACCCACTTGGTGGGATAAGGCC
CCCCGCAGAACACGGGTTCAATAGGTCAGACCTGGAAGCTCAGTAATGGGTGT
AGGGAACTGGTGCTAACCGGCCGAAAACTTACAACACCCTCCCTTTTGGAAAA
GGGAGGCAAAAACAAACTCGCAACCACATCCGTTCACGGCGCTAGCCGTGCGT
CCACACCCCCCACCAGAACAAATTTGCATAGAGTTACGGCGGCCACAGCGGC
3´

SEQ.ID.NO.47 (M. tuberculosis, Patientenisolat)
MTB-P08.seq (263bp)
5´CGGGAAACCTTCTCCAAGATCAGGTTTCTCACCCACTTGGTGGGATAAGGCC
CCCCGCAGAACACGGGTTCAATAGGTCAGACCTGGAAGCTCAGTAATGGGTGT
AGGGAACTGGTGCTAACCGGCCGAAAACTTACAACACCCTCCCTTTTGGAAAA
GGGAGGCAAAAACAAACTCGCAACCACATCCGTTCACGGCGCTAGCCGTGCGT
CCACACCCCCCACCAGAACAAATTTGCATAGAGTTACGGCGGCCACAGCGGC
3´

SEQ.ID.NO.48 (M. tuberculosis, Patientenisolat)
MTB-P10.seq (263bp)
5´CGGGAAACCTTCTCCAAGATCAGGTTTCTCACCCACTTGGTGGGATAAGGCC
CCCCGCAGAACACGGGTTCAATAGGTCAGACCTGGAAGCTCAGTAATGGGTGT
AGGGAACTGGTGCTAACCGGCCGAAAACTTACAACACCCTCCCTTTTGGAAAA
GGGAGGCAAAAACAAACTCGCAACCACATCCGTTCACGGCGCTAGCCGTGCGT
CCACACCCCCCACCAGAACAAATTTGCATAGAGTTACGGCGGCCACAGCGGC
3´

SEQ.ID.NO.49 (M. tuberculosis, Patientenisolat)
MTB-P14.seq (263bp)
5´CGGGAAACCTTCTCCAAGATCAGGTTTCTCACCCACTTGGTGGGATAAGGCC
CCCCGCAGAACACGGGTTCAATAGGTCAGACCTGGAAGCTCAGTAATGGGTGT
AGGGAACTGGTGCTAACCGGCCGAAAACTTACAACACCCTCCCTTTTGGAAAA
GGGAGGCAAAAACAAACTCGCAACCACATCCGTTCACGGCGCTAGCCGTGCGT
CCACACCCCCCACCAGAACAAATTTGCATAGAGTTACGGCGGCCACAGCGGC
3´

SEQ.ID.NO.50 (M. phlei)
PHLEI.seq (251bp)
5´CGGGAAACCTTCTCCAAGATCAGGCTTTCACCCTTTAGGAGGGATAAGGCC
CCCCGCAGACCACGGGATCGATAGACCAGACCTGCACGCACAGCAATGTGTTC
AGGGAACTGGCATTAACCGGCCGAAAACTTACACACAACGTTGCAACCACATC

CAACAGTCACCATGATTGCACCCCACCACCAAAACATTCCCCCCCCCAACAAC
AACTGGTGCACTTAAATAGAGTTACGGCGGCCACAGCGNC 3′

SEQ.ID.NO.51 (M. smegmatis)
SMEGM.seq (305bp)
5´CGGGAAACCTTCTCCAAGATCAGCTTTTCACCCATGGGGAGGGATAAGGCCC
CCCGCAGAACACTGAATTATAGGTCAGACCTGGAAGCTAGTAATACGGTTGCA
GGGAGAACTGGCACTAACCGGCCGAAAACTTACAACACCCCATAATCGTTGTA
GAAGAAACATTGACGYACCGCGACTCGGGCAACCACATDCCACGGATGATAC
AAACCGATACAGCCCCACCACCAAAACAAAGCCCACAAGTTTGCTCTCATGTG
GGTCACCAAATAAGAGAATAGAGTTACGGCGGTCATAGCGGA 3´

SEQ.ID.NO.52 (M. szulgai, ATCC 35799)
SZU35799.seq (245bp)
5´CGGGAAACCTTCTCCAAGATCAGGCTTCTCACCCTTTTAGAGGGATATAGGC
CCCTCCGCAGAACACGGGATCGATAGGCCAGACCTGGAAGCTCAGCAATGAG
TGAAGGGAACTGGCACTAACCGGCCGAAAACTTACCAACACAACAAATCGCA
ACCACAATCCATTTCACGGCTTATAGCCGTAAAACACCGCACCCCGCCACCAG
TACAAAYYTAAATAGAGTTACGGCGGCCACAGCGAC 3´

SEQ.ID.NO.53 (M. szulgai)
SZULGAI.seq (246bp)
5´CGGGAAACCTTCTCCAAGATCAGGCTTCTCACCCTTTTAGAGGGATATAGGC
CCCTCCGCAGAACACGGGATCGATAGGCCAGACCTGGAAGCTCAGCAATGAG
TGAAGGGAACTGGCACTAACCGGCCGAAAACTTACCAACACAACAAATCGCA
ACCACAATCCATTTCACGGCTTGATAGCCGTAAAACACCGCACCCCGCCACCA
GTACAAATTTAAATAGAGTTACGGCGGCCACAGCGAC 3´

SEQ.ID.NO.54 (M. terrae)
TERRAE.seq (263bp)
5´CGGGAAACCTTCTCCAAGATCAGGTTTCTCACCCACTTGGTGGGATAAGGCC
CCCCGCAGAACACGGGTTCAATAGGTCAGACCTGGAAGCTCAGTAATGGGTGT

AGGGAACTGGTGCTAACCGGCCGAAAAYTTACAACMCCCTCCCTTTTGGAAAA
GGGAGGCAAAAACAAMCTCGHAACCACATCCGTTCACGGCGCTAGCCGTGCG
TCCACACCCCCCACCAGAACAAATTTGCATAGAGTTACGGCGGCCACAGCGNC
3´

SEQ.ID.NO.55 (M. xenopi)
XENOPI.seq (263bp)
5´CGGGAAACCTTCTCCAAGATCAGGTTTCTCACCCACTTGGTGGGATAAGGCC
CCCCGCAGAACACGGGTTCAATAGGTCAGACCTGGAAGCTCAGTAATGGGTGT
AGGGAACTGGTGCTAACCGGCCGAAAACTTACAACACCCTCCCTTTTGGAAAA
GGGAGGCAAAAACAAACTCGCAACCACATCCGTTCACGGCGCTAGCCGTGCGT
CCACACCCCCCACCAGAACAAATTTGCATAGAGTTACGGCGGCCACAGCGGC
3´

SEQ.ID.NO.72 (Mehrheitssequenz der 23S-5S-Spacerregion aller untersuchten
Isolate des MAI-Komplexes)
5´CGGGAAACCTTCTCCAAGATCAGGTTTCTCACCCTTTTAGAGGGATAAGGCC
CCCCGCAGACCACGGGATTGATAGGCCAGACCTGGAAGCTCAGTAATGAGTGC
AGGGAACTGGCACTAACCGGCCGAAAACTTACCAACACACAACTCGCAACCA
CGGTCCATTTCCGCGGCAATTCGCCGCTGGAAACTCGGGCACCACACCCCCCA
CCGAAACAAATTTAAATAGAGTTACGGCGGCCACAGCGAC 3´

## Patentansprüche

1. Verfahren zur Amplifikation von Nukleinsäuren der Gattung Mycobacterium, **dadurch gekennzeichnet, daß** eine Nukleinsäure amplifiziert wird, die eine Länge von mehr als 50 Nukleotiden hat, die zu mehr als 90 % homolog oder komplementär zu aufeinanderfolgenden Basen der SEQ.ID.NO.1 oder einer der Sequenzen SEQ.ID.NO.5 bis SEQ.ID.NO.55 ist.

2. Verfahren gemäß Anspruch 1, **dadurch gekennzeichnet, daß** die Amplifikation unter Verwendung mindestens eines gattungsspezifischen Primers durchgeführt wird, der mit einem Strang in der 23S-Region oder in der 5S-Region von Mycobakterien hybridisiert.

3. Verfahren zum Nachweis von Bakterien einer Gruppe der Gattung Mycobakterien in einer Probe, **dadurch gekennzeichnet, daß** die Probe einer mycobakterienspezifischen Nukleinsäureamplifikation unterzogen wird und die Mitglieder der Gruppe mit einer gruppenspezifischen Sonde nachgewiesen werden.

4. Verfahren gemäß Anspruch 3, **dadurch gekennzeichnet, daß** die Gruppe der MTB-Komplex oder der MAI-Komplex ist.

5. Verfahren zum Nachweis von Bakterien einer Spezies der Gattung Mycobakterien in einer Probe, **dadurch gekennzeichnet, daß** eine Sonde eingesetzt wird, die ein mindestens 8 Basen langer, fortlaufender Teil einer Sequenz ist, die eine Homologie oder Komplementarität von mehr als 90 % zu SEQ.ID.NO.1 oder einer der Sequenzen SEQ.ID.NO.5 bis SEQ.ID.NO.55 aufweist.

6. Verfahren gemäß Anspruch 5, **dadurch gekennzeichnet, daß** die Sonde ein Primer ist.

7. Verfahren gemäß Anspruch 6, **dadurch gekennzeichnet, daß** mindestens zwei Primer eingesetzt werden, die jeweils einen mindestens 10 Basen langen, fortlaufenden Teil einer Sequenz enthalten, die eine Homologie oder Komplementarität von mehr als 90 % zu SEQ.ID.NO.1 1 oder einer der Sequenzen SEQ.ID.NO.5-SEQ.ID.NO.55 aufweist.

8. Verfahren zum Nachweis von Bakterien einer Subspezies der Gattung Mycobacterium aus einer Probe, **dadurch gekennzeichnet, daß** eine Sonde eingesetzt wird, die ein mindestens 12 Basen langer, fortlaufender Teil einer Sequenz ist, die eine Homologie oder Komplementarität von mehr als 90 % zu einer der SEQ.ID.NO. 5 bis 55 aufweist.

9. Verfahren gemäß Anspruch 3, **dadurch gekennzeichnet, daß** eine Sonde verwendet wird, die zu mehr als 90 % homolog oder komplementär zu mindestens einer der Sequenzen SEQ.ID.N0.5 bis 55 ist.

10. Verfahren gemäß einem der Ansprüche 5 bis 9, **dadurch gekennzeichnet, daß** die Probe vorher den Bedingungen einer Mycobakterien, Mycobakteriengruppen- oder Mycobakterienspezies-spezifischen Amplifikation unterzogen wird.

11. Nukleinsäuresonde zum Nachweis von Spezies oder Subspezies der Bakterien der Gattung Mycobacterium, welche eine fortlaufende Sequenz von mehr als 12 Basen aufweist, die zu mindestens 90 % homolog zu mindestens einer der Sequenzen SEQ.ID.NO.5 bis 55 ist oder die zu mindestens 90 % komplementär zu einem Teil der SEQ.ID.NO.1 ist.

12. Sonde gemäß Anspruch 11, **dadurch gekennzeichnet, daß** die Sequenz ausgewählt wird aus den Sequenzen der SEQ.ID.NO.1 bis 55.

13. Set von Primern zur Amplifikation von genomischen Mycobakteriennukleinsäuren, enthaltend mindestens zwei Sonden gemäß Anspruch 11 oder 12.

14. Sonde gemäß Anspruch 12, **dadurch gekennzeichnet, daß** sie subspeziesspezifisch ist.

15. Doppelsträngige Mycobakterien-spezifische Nukleinsäure enthaltend eine Nukleinsäure die eine Länge von mehr als 50 Nukleotiden hat und die zu mehr als 90 % homolog oder komplementär zu aufeinanderfolgenden Basen der SEQ.ID.NO.1 oder einer der Sequenzen SEQ.ID.NO.5 bis 55 ist.

16. Reagenzkit zum Nachweis von Mycobakterien-Spezies enthaltend
- mindestens eine Mycobakterien-gattungs- oder -gruppenspezifische Sonde und
- mindestens eine Mycobakterien-spezies- oder -subspeziesspezifische Sonde.
